# EUROPEAN PATENT APPLICATION

(11) **EP 2 298 750 A1**
(43) Date of publication of application: **23.03.2011**
(21) Application number: 09758257.1
(22) Date of filing: 28.05.2009
(51) Int. Cl.: C07D 261/12, A61K 31/42, A61K 31/4439, A61P 3/04, A61P 3/06, A61P 3/10, A61P 43/00, C07D 413/04, C07D 413/12

(54) **NOVEL ISOXAZOLE DERIVATIVE**

(30) Priority: 02.06.2008 JP 2008144851
(71) Applicant: MSD K.K., Chiyoda-ku Tokyo 102-8667 (JP)
(72) Inventor: ISHIKAWA Makoto, Tokyo 102-8667 (JP); HAKETA Tasuku, Tokyo 102-8667 (JP); NAKAMA Chisato, Tokyo 102-8667 (JP); NISHIMURA Teruyuki, Tokyo 102-8667 (JP); SHIBATA Jun, Tokyo 102-8667 (JP); SHIMAMURA Tadashi, Tokyo 102-8667 (JP); YAMAKAWA Takeru, Tokyo 102-8667 (JP)
(74) Representative: Buchan, Gavin MacNicol
(86) International application number: PCT/JP2009/059758
(87) International publication number: WO 2009/147990

(57) **Abstract**

Disclosed is a compound represented by formula (I) or a pharmaceutically acceptable salt thereof, which has an agonistic activity on GPR120 and is therefore useful for the treatment of diabetes, obesity or hyperlipemia. [In formula (I), the A represents a phenyl group which may be substituted by a lower alkoxy group or the like, or the like; the B represents a bivalent group produced by removing two hydrogen atoms from a benzene ring which may be substituted by a halogen atom or the like, or the like; X represents a lower alkylene group having 2 to 4 carbon atoms in its main chain or the like, wherein a carbon atom constituting the main chain may be substituted by an oxygen atom or the like; and Y represents a hydrogen atom or the like.]

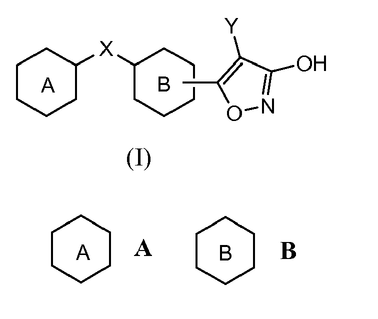

## Description

### FIELD OF THE INVENTION

The present invention relates to isoxazole derivatives that are useful in the pharmaceutical field. The compounds act as GPR120 receptor (14273) function regulating agents, which are useful as drugs for treating and/or preventing diabetes mellitus, obesity and hyperlipidemia.

### BACKGROUND OF THE INVENTION

GPR120, a G protein-coupled receptor, causes intracellular signaling through binding with unsaturated long chain fatty acid, such as alpha-linoleic acid, to induce various biological reactions. Actions of GPR120 and its ligand have been reported to promote secretion of GLP-1 (glucagon-like-peptide-1) having the function of reducing a blood glucose level in the gastrointestinal cell lines. GLP-1, which is a peptide hormone released from L cells which are enteroendocrine cells present in the ileum, the large intestine and the like, has been found to induce insulin secretion depending on a blood glucose level. Accordingly, compounds having the action of promoting GLP-1 secretion are expected as agents for treating diabetes mellitus that allow avoidance of the risk of hypoglycemia due to drug overdosage. GLP-1 is also suggested to be efficacious for delaying the apoptosis of beta cells in type II diabetes mellitus or prolonging the efficacy of islet cell transplantation against type I diabetes mellitus because of having the action of inducing pancreatic beta-cell growth and differentiation from stem cells. GPR120 is known to be also expressed in adipocytes. GPR120 has been found to be increasingly expressed by adipose differentiation induction. In addition, actions of GPR120 and its ligand have been reported to suppress lipolysis in adipose-differentiated cells. A high blood lipid level is known to be one of the causes of insulin resistance. Suppression of lipolysis by a GPR120 agonist is thus expected to decrease the level of free fatty acid in blood to normalize a blood lipid level, resulting in improvement in insulin resistance. Furthermore, GPR120 is also expressed in the pituitary gland, and a GPR120 ligand is reported to suppress adrenocorticotropic hormone secretion. Adrenocorticotropic hormone promotes glucocorticoid secretion downstream thereof to induce action such as promotion of glyconeogenesis in the liver, inhibitory action against glucose uptake in muscle and peripheral tissue, lipolysis in adipose tissue or release of fatty acid or glycerol. Accordingly, GPR120 is considered to exhibit hypoglycemic action or blood lipid lowering action via suppression action against adrenocorticotropic hormone secretion even in the center. In light of the above description, a compound having GPR120 agonist activity is considered to be extremely useful as an agent for treating and/or preventing diabetes mellitus, obesity and hyperlipidemia.

Compounds related structurally to a compound according to an embodiment of the present invention include, e.g., a compound represented by the following formula: 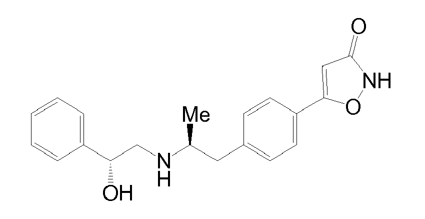 which is described (see patent reference 1).

In the compound, a main chain via which phenyl and isoxazol-5-yl-phenyl are bound is 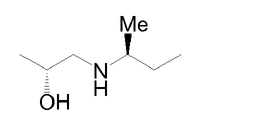 and the compound differs from a compound according to an embodiment of the present invention.
Patent reference 1: Japanese Patent Laid-Open No. 62-175458
Non-patent reference 1: nature medicine, vol 11, No 1, page 90-941 (2005)

### SUMMARY OF THE INVENTION

It is desirable to provide a novel isoxazole derivative having a GPR120 (14273) function regulating action.

We, the present inventors have assiduously studied to develop a compound having a GPR120 (14273) function regulating action, particularly having an agonist action, and found that the compound according to an embodiment of the present invention is efficacious as the compound having the GPR120 (14273) function regulating action, and the invention was thus accomplished based on such findings.

Specifically, the present invention relates to a compound represented by the formula (I): 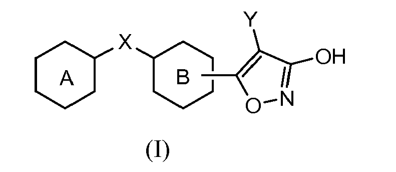 or a pharmaceutically acceptable salt thereof, wherein: 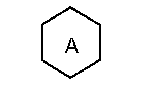 represents phenyl, 5- or 6-membered heteroaryl, or fused ring with phenyl or 5- or 6-membered heteroaryl said phenyl, 5- or 6-membered heteroaryl, or fused ring with phenyl or 5- or 6-membered heteroaryl, is optionally substituted with 1-4 same or different groups selected from the group consisting of:
lower alkoxy optionally substituted 1-3 halogen atoms,
lower alkoxycarbonyl,
lower alkenyloxy,
lower alkynyloxy,
lower alkylthio optionally substituted with 1-3 halogen atoms,
halo(lower)alkyloxy, cycloalkyloxy,
lower alkyl optionally substituted with 1-3 halogen atoms,
lower alkenyl,
lower alkynyl,
cycloalkylthio,
lower alkylamino,
lower alkenylamino,
lower alkynylamino,
lower haloalkylamino,
cycloalkylamino,
nitro,
halogen,
cyano,
lower alkylsulfonyl,
lower alkenylsulfonyl,
lower alkynylsulfonyl,
phenoxy,
phenyl,
5- or 6-membered heteroaryloxy,
5- or 6-membered heteroaryl,
5- or 6-membered heteroarylalkyloxy, and
Arylalkyloxy; 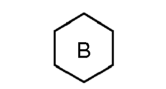 represents a divalent group, in which two hydrogen atoms are removed from a benzene, pyridine, pyrazine, pyrimidine or pyridazine ring, and which is optionally substituted with 1-4 same or different groups selected from the group consisting of:
   halogen,
   lower alkyl, and
   lower alkoxy;
   X is lower alkylene having a main chain including 2-4 carbon atoms, wherein one or two of the carbon atoms constituting the main chain is optionally substituted with oxygen, sulfur or nitrogen,
   said lower alkylene is substituted with 1-3 same or different groups selected from the group consisting of:
      (1) amino optionally substituted with lower alkyl, lower alkylsulfonyl and lower alkanoyl,
      (2) halogen,
      (3) lower alkenyl,
      (4) lower alkyl, when identical carbon atoms constituting the lower alkylene are substituted with two lower alkyl groups, the lower alkyl groups may together form 3- to 7-membered aliphatic rings , and one or two of carbon atoms constituting the aliphatic rings is optionally substituted with nitrogen, sulfur or oxygen,
      (5) lower alkynyl,
      (6) lower alkylidene,
      (7) imino,
      (8) arylalkyl,
      (9) 5- or 6-membered heteroarylalkyl having 1-4 hetero atoms selected from the group consisting of nitrogen, sulfur and oxygen atoms in a ring,
      (10) heteroaryl having 1-4 hetero atoms selected from the group consisting of nitrogen, sulfur and oxygen atoms in a ring,
      (11) arylalkyloxy, and
      (12) 5- or 6-membered heteroarylalkyloxy having 1-4 hetero atoms selected from the group consisting of nitrogen, sulfur and oxygen atoms in a ring;
         said lower alkyl, lower alkynyl and lower alkylidene is optionally substituted with 1-3 halogen atoms or hydroxy or lower alkoxy groups;
         said imino is optionally substituted with 1-3 lower alkyl, lower alkylsulfonyl, cyano, nitro and lower alkanoyl groups;
         said lower alkylene may have one or two double or triple bonds in a main chain and, in addition, the lower alkylene is optionally substituted with 1-3 lower alkoxy, oxo or hydroxy groups;
         X together with 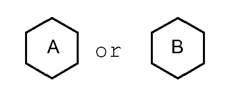 may form 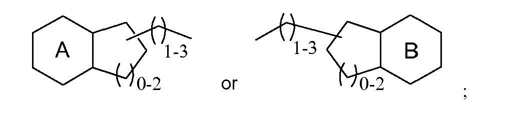 one or two of carbon atoms constituting 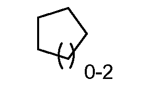 is optionally substituted with nitrogen, sulfur or oxygen and have one or two double bonds in a ring; one carbon atom constituting 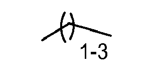 is optionally substituted with nitrogen, sulfur or oxygen; and
         Y represents hydrogen, lower alkyl optionally substituted with 1-3 same or different lower alkoxy groups and halogen atoms, lower alkoxy or halogen.

The present invention also relates to a GPR120 function regulating agent containing a compound represented by the formula (I) or a pharmaceutically acceptable salt thereof as an active ingredient. Particularly, the present invention relates to a GPR (14273) 120 agonist containing a compound represented by the formula (I) or a pharmaceutically acceptable salt thereof as an active ingredient.

The present invention also relates to an agent for treating diabetes mellitus, obesity or hyperlipidemia, containing a compound represented by the formula (I) or a pharmaceutically acceptable salt thereof as an active ingredient.

Furthermore, the present invention relates to a pharmaceutical composition containing a compound represented by the formula (I) and a pharmaceutically acceptable carrier.

A compound (I) according to an embodiment of the present invention or a pharmaceutically acceptable salt thereof has a strong GPR120 (14273) function regulating action, particularly an agonist action, and is useful for treating and/or preventing diabetes mellitus, obesity or hyperlipidemia.

### DETAILED DESCRIPTION OF THE INVENTION

The meanings of terms as used herein are described below, and a compound according to an embodiment of the present invention is described in further detail.

The term "halogen" includes, for example, fluorine, chlorine, bromine and iodine.

The term "lower alkyl" means linear or branched C₁₋₆ alkyl and includes, for examples, methyl, ethyl, propyl, isopropyl, butyl, isobutyl, sec-butyl, tert-butyl, pentyl, isoamyl, neopentyl, isopentyl, 1,1-dimethylpropyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,2,2-trimethylpropyl and 1-ethyl-2-methylpropyl.

The term "lower alkoxy" means a group, in which a hydrogen atom of hydroxy is substituted with said lower alkyl, and includes, for examples, methoxy, ethoxy, propoxy, isopropoxy, butoxy, sec-butoxy, tert-butoxy, pentyloxy, isopentyloxy, hexyloxy and isohexyloxy.

The term "cycloalkyl" means a cycloalkyl group having 3 to 7 carbon atoms (C₃₋₇ cycloalkyl) and specifically includes, for examples, cyclopropyl, cyclobutyl, cyclopentyl, cyclohexyl and cycloheptyl.

The term "lower alkylthio" means a group, in which a hydrogen atom of thiol is substituted with said lower alkyl, and specifically includes, for example, methylthio, ethylthio, n-propylthio, isopropylthio, butylthio and isobutylthio.

The term "cycloalkyloxy" means a group, in which a hydrogen atom of hydroxy is substituted with the above-defined cycloalkyl, and specifically includes, for example, cyclopropyloxy, cyclobutyloxy, cyclopentyloxy and cyclohexyloxy.

The term "cycloalkylthio" means a group, in which a hydrogen atom of thiol is substituted with said cycloalkyl, and specifically includes, for example, cyclopropylthio, cyclobutylthio, cyclopentylthio and cyclohexylthio.

The term "lower alkylamino" refers to a group, in which one or two of hydrogen atoms of amino are substituted with the above-defined same or different lower alkyl, and specifically includes, for example, methylamino, ethylamino, n-propylamino, isopropylamino, dimethylamino, diethylamino and ethylmethylamino.

The term "cycloalkylamino" means a group, in which one or two of hydrogen atoms of amino are substituted with the above-defined same or different cycloalkyl, and specifically includes, for example, cyclopropylamino, cyclobutylamino, cyclopentylamino and cyclohexylamino.

The term "lower alkylsulfonyl" includes, for example, methylsulfonyl, ethylsulfonyl, propylsulfonyl and isopropylsulfonyl.

The term "lower alkenyl" includes, for example, vinyl, propenyl, isopropenyl, 2-buten-1-yl and 4-penten-1-yl.

The term "lower alkynyl" includes, for example, 2-butyn-1-yl and 4-pentyn-1-yl.

The term "lower alkenyloxy" includes, for example, vinyloxy, propenyloxy, isopropenyloxy, 2-buten-1-yloxy and 4-penten-1-yloxy.

The term "lower alkynyloxy" includes, for example, 2-butyn-1-yloxy and 4-pentyn-1-yloxy.

The term "halo(lower)alkyl" means alkyl, of which the alkyl portion has 1-6 carbon atoms and straight or branched, and includes, for example, fluoromethyl, trifluoromethyl, chloromethyl, 2,2,2-trifluoroethyl, 3-chloropropyl, 3-fluoropropyl, 4-chlorobutyl, 4-fluorobutyl, 5-chloropentyl, 6-chlorohexyl and 6-fluorohexyl.

The term "halo(lower)alkyloxy" includes, for example, fluoromethoxy, trifluoromethoxy, chloromethoxy, 2,2,2-trifluoromethoxy, 3-chloropropyloxy, 3-fluoropropyloxy, 4-chlorobutyloxy, 4-fluorobutyloxy, 5-chloropentyloxy, 6-chlorohexyloxy and 6-fluorohexyloxy.

The term "lower alkenylamino" includes, for example, vinylamino, propenylamino, isopropenylamino, 2-buten-1-ylamino and 4-penten-1-ylamino.

The term "lower alkynylamino" includes, for example, 2-butyn-1-ylamino and 4-pentyn-1-ylamino.

The term "lower halo(lower)alkylamino" includes, for example, fluoromethylamino, trifluoromethylamino, chloromethylamino, 2,2,2-trifluoromethylamino, 3-chloropropylamino, 3-fluoropropylamino, 4-chlorobutylamino, 4-fluorobutylamino, 5-chloropentylamino, 6-chlorohexylamino and 6-fluorohexylamino.

The term "lower alkenylsulfonyl" includes, for example, vinylsulfonyl, propenylsulfonyl, isopropenylsulfonyl, 2-buten-1-ylsulfonyl and 4-penten-1-ylsulfonyl.

The term "lower alkynylsulfonyl" includes, for example, 2-butyn-1-ylsulfonyl and 4-pentyn-1-ylsulfonyl.

The term "lower alkylidene" means C₁₋₆ alkylidene and specifically includes, for example, methylidyne, ethylidene, propylidene, butylidene, pentylidene and hexylidene.

Each symbol used in the formula (I) in accordance with an embodiment of the present invention 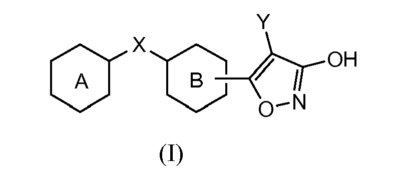 is specifically described.

Of the compounds represented by the formula (I), a compound represented by the formula (I-1): 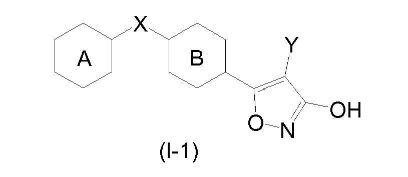 is preferred.

The formula: 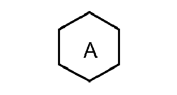 represents phenyl, 5- or 6-membered heteroaryl, or fused ring with phenyl or 5- or 6-membered heteroaryl said phenyl, 5- or 6-membered heteroaryl, or fused ring with said phenyl or 5- or 6-membered heteroaryl, is optionally substituted with 1-4 same or different groups selected from the group consisting of:
lower alkoxy optionally substituted 1-3 halogen atoms,
lower alkoxycarbonyl,
lower alkenyloxy,
lower alkynyloxy,
lower alkylthio optionally substituted with 1-3 halogen atoms, halo(lower)alkyloxy, cycloalkyloxy,
lower alkyl optionally substituted with 1-3 halogen atoms, lower alkenyl,
lower alkynyl,
cycloalkylthio,
lower alkylamino,
lower alkenylamino,
lower alkynylamino,
lower haloalkylamino,
cycloalkylamino,
nitro,
halogen,
cyano,
lower alkylsulfonyl,
lower alkenylsulfonyl,
lower alkynylsulfonyl,
phenoxy,
phenyl,
5- or 6-membered heteroaryloxy,
5- or 6-membered heteroaryl,
5- or 6-membered heteroarylalkyloxy, and arylalkyloxy.

The term "5- or 6-membered heteroaryl" represented by the formula: 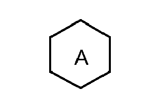 means 5- or 6-membered heteroaryl having 1-4 same or different hetero atoms selected from the group consisting of nitrogen, sulfur and oxygen atoms.

The term "5- or 6-membered heteroaryl" includes, for example, pyridinyl, oxazolyl, isoxazolyl, thiazolyl, thiadiazolyl, imidazolyl, tetrazolyl or pyrazolyl.

The term "fused ring with phenyl or 5- or 6-membered heteroaryl" represented by the formula: 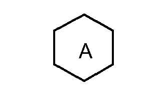 includes, for example, isobenzofuranyl (such as 1-benzofuranyl), chromenyl (such as 2H-chromen-3-yl), benzothienyl (such as 2-benzothienyl), indolizinyl (such as 2-indolizinyl or 3-indolizinyl), isoindolyl (such as 1-isoindolyl), 3H-indolyl (such as 3H-indol-2-yl), indolyl (such as 2-indolyl), 1H-indazolyl (such as 1H-indazol-3-yl), purinyl (such as 8-purinyl), isoquinolyl (such as 1-isoquinolyl or 3-isoquinolyl), quinolyl (such as 2-quinolyl or 3-quinolyl), phthalazyl (such as 1-phthalazyl), naphthyridinyl (such as 1,8-naphthyridin-2-yl), quinoxalinyl (such as 2-quinoxalinyl), quinazolinyl (such as 2-quinazolinyl), cinnolinyl (such as 3-cinnolinyl), isochromanyl (such as 3-isochromanyl), indolinyl (such as 2-indolinyl), isoindolinyl (such as 1-isoindolinyl), 1,2,3,4-tetrahydro-2-quinolyl and 1,2,3,4-tetrahydro-3-isoquinolyl.

A group represented by the formula: 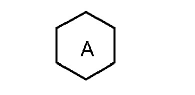 is optionally substituted with 1-4 same or different groups selected from the group consisting of lower alkoxy, lower alkenyloxy, lower alkynyloxy, lower haloalkyloxy, cycloalkyloxy, lower alkylthio, lower alkyl, lower alkenyl, lower alkynyl, lower haloalkyl, cycloalkylthio, lower alkylamino, lower alkenylamino, lower alkynylamino, lower haloalkylamino, cycloalkylamino, nitro, halogen, cyano, lower alkylsulfonyl, lower alkenylsulfonyl, lower alkynylsulfonyl, phenoxy, phenyl, 5- or 6-membered heteroaryloxy and 5- or 6-membered heteroaryl.

The term "lower alkoxy" of the substituent means an identical group as the term "lower alkoxy" defined above and specifically encompasses, for example, methoxy, ethoxy, propoxy and isopropoxy.

The term "lower alkenyloxy" of the substituent means an identical group as the term "lower alkenyloxy" defined above.

The term "lower alkynyloxy" of the substituent means an identical group as the term "lower alkynyloxy" defined above.

The term "halo(lower)alkoxy" of the substituent means a lower alkoxy substituted with 1-3 same or different halogen atoms.

The term "cycloalkyloxy" of the substituent means an identical group as the term "cycloalkyloxy" defined above.

The term "lower alkylthio" of the substituent means a group as the term "lower alkylthio" defined above.

The term "lower alkylamino" of the substituent means an identical group as the term "lower alkylamino" defined above.

The term "lower alkenylamino" of the substituent means an identical group as the term "lower alkenylamino" defined above.

The term "lower alkynylamino" of the substituent means an identical group as the term "lower alkynylamino" defined above.

The term "halo(lower)alkylamino" of the substituent refers to lower alkylamino substituted with 1-3 same or different halogen atoms.

The term "cycloalkylamino" of the substituent means an identical group as the term "cycloalkylamino" defined above.

The term "halogen" of the substituent means an identical group as the term "halogen" defined above.

The term "lower alkylsulfonyl" of the substituent means an identical group as the term "lower alkylsulfonyl" defined above.

The term "5- or 6-membered heteroaryl" of the substituent means heteroaryl having 1-4 hetero atoms selected from the group consisting of nitrogen, sulfur and oxygen atoms in a ring and specifically includes, for example, pyridinyl, oxazolyl, isoxazolyl, thiazolyl, thiadiazolyl, imidazolyl, tetrazolyl and pyrazolyl.

The term "5- or 6-membered heteroaryloxy" of the substituent means a group in which heteroaryl having 1-4 hetero atoms selected from the group consisting of nitrogen, sulfur and oxygen atoms in a ring and oxy are bonded. 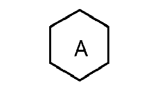 is preferably phenyl or pyridinyl substituted with 1-2 same or different groups selected from the group consisting of:
lower alkoxy optionally substituted with 1-3 same or different halogen atoms,
C₃₋₇ cycloalkyloxy,
lower alkyl optionally substituted with 1-3 same or different halogen atoms,
lower alkenyl, and
halogen atoms;
more preferably a group selected from the group consisting of: 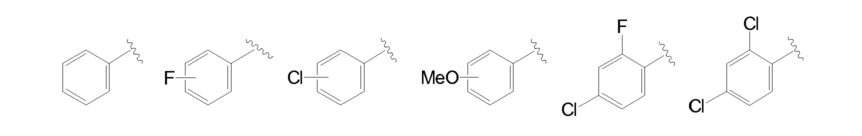 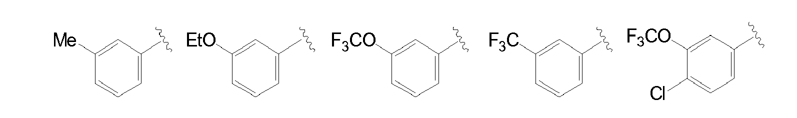 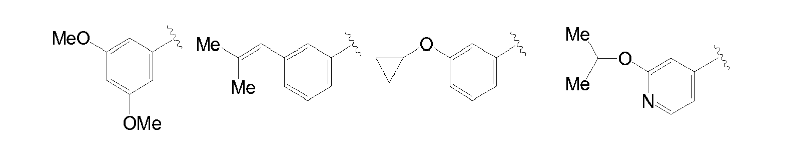 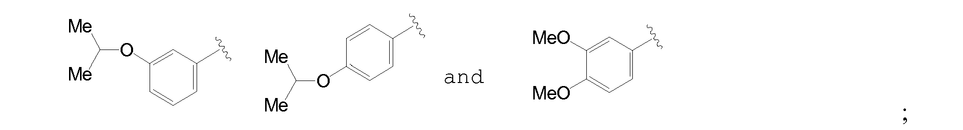 further preferably a group selected from the group consisting of:

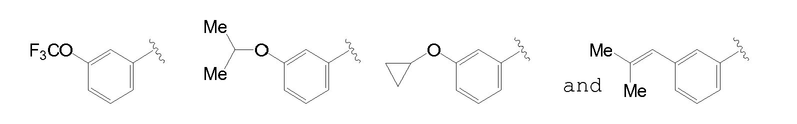

The formula: 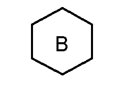 represents a divalent group, in which two hydrogen atoms are removed from a benzene, pyridine, pyrazine, pyrimidine or pyridazine ring, said 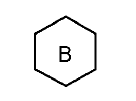 is optionally substituted with 1-4 same or different groups selected from the group consisting of:
halogen,
lower alkyl,
and lower alkoxy.

The divalent group, in which two hydrogen atoms are removed from a benzene, pyridine, pyrazine, pyrimidine or pyridazine ring, means a group represented by 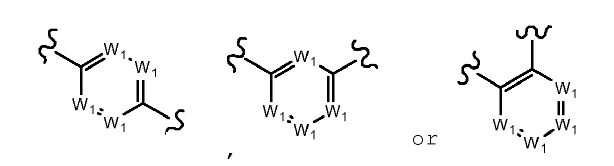 wherein all the symbols W₁ are carbon atoms; or one or two of symbols W₁ are nitrogen atoms and the other symbols W₁ are carbon atoms optionally substituted with 1-4 groups selected from the group consisting of halogen, lower alkyl and lower alkoxy, 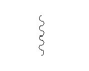 represents a binding site with X or 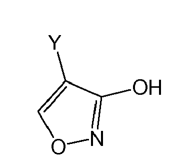 in the formula.

The term "halogen" of the substituent means an identical group as the term "halogen" defined above.

The term "lower alkyl" of the substituent means an identical group as the term "lower alkyl" defined above.

The term "lower alkoxy" of the substituent means an identical group as the term "lower alkoxy" defined above. 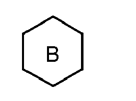 is preferably a divalent group, in which two hydrogen atoms are removed from a benzene or pyridin ring,
said divalent group is optionally substituted with 1-4 same or different groups selected from the group consisting of halogen, lower alkyl and lower alkoxy;
more preferably a group selected from the group consisting of: 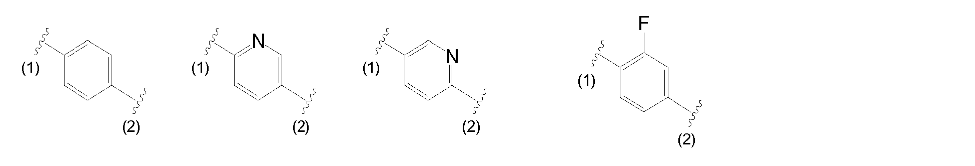 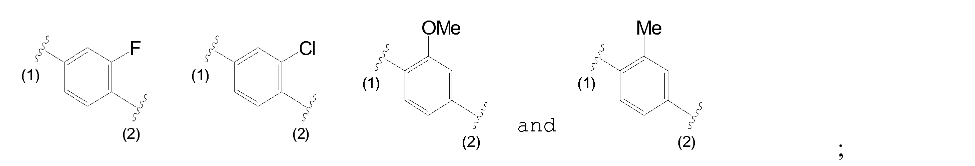 further preferably a group selected from the group consisting of: 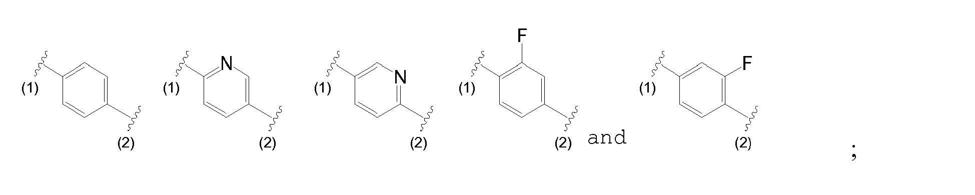 wherein 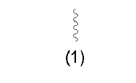 represents a binding site with X; and 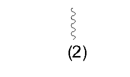 represents a binding site with 3-hydroxy-isoxazolyl.

X means lower alkylene having a main chain containing 2-4 carbon atoms.

X has a main chain constituted of carbon atoms of which one or two is optionally substituted with nitrogen, sulfur or oxygen.

X is substituted with 1-3 same or different groups selected from the group consisting of:
(1) amino optionally substituted with 1-3 groups of lower alkyl, lower alkylsulfonyl and lower alkanoyl,
(2) halogen,
(3) lower alkenyl,
(4) lower alkyl,
   when identical carbon atoms constituting X are substituted with two lower alkyl groups, the lower alkyl groups may together form 3- to 7-membered aliphatic rings, and
   one or two of carbon atoms constituting the aliphatic rings is optionally substituted with nitrogen, sulfur or oxygen,
(5) lower alkynyl,
(6) lower alkylidene,
(7) imino,
(8) arylalkyl,
(9) heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring.

The term "amino optionally substituted with 1-3 groups of lower alkyl, lower alkylsulfonyl and lower alkanoyl" of the substituent specifically includes, for example, methylamino, ethylamino, propylamino, isopropylamino, methylsulfonylamino, ethylsulfonylamino, propylsulfonylamino, isopropylsulfonylamino, acetylamino, ethylcarbonylamino, propylcarbonylamino and isopropylcarbonylamino.

The term "halogen" of the substituent means an identical group as the term "halogen" defined above.

The term "lower alkenyl" of the substituent means an identical group as the term "lower alkenyl" defined above.

The term "lower alkyl" of the substituent means an identical group as the term "lower alkyl" defined above.

When identical carbon atoms constituting the symbol X are substituted with two lower alkyl groups, the lower alkyl groups may together form 3- to 7-membered aliphatic rings.

Examples of the 3- to 7-membered aliphatic rings specifically include cyclopropyl, cyclobutyl, cyclopentyl, cyclohexyl and cycloheptyl. One or two of carbon atoms constituting the 3- to 7-membered aliphatic rings is optionally substituted with nitrogen, sulfur or oxygen.

The term "lower alkynyl" of the substituent refers to an identical group as the term "lower alkynyl" defined above.

The term "lower alkylidene" of the substituent refers to an identical group as the term "lower alkylidene" defined above.

The term "arylalkyl" of the substituent specifically includes, for example, benzyl.

The term "5- or 6-membered heteroaryl having 1-4 hetero atoms selected from the group consisting of nitrogen, sulfur and oxygen atoms in a ring" of the substituent means an identical group as the term "heteroaryl" defined above.

The term "heteroarylalkyl" of the substituent means a group in which "heteroaryl" defined above and "lower alkyl" defined above are bonded.

The term "arylalkyloxy" of the substituent specifically includes, for example, benzyloxy.

The term "heteroarylalkyloxy" of the substituent means a group in which "heteroaryl" defined above and "lower alkoxy" defined above are bonded.

The lower alkyl, lower alkynyl and lower alkylidene of the substituents is optionally substituted with 1-3 halogen atoms or hydroxy or lower alkoxy groups.

The imino of the substituent is optionally substituted with 1-3 lower alkyl, lower alkylsulfonyl, cyano, nitro or lower alkanoyl.

X has one or two double bonds or triple bonds.

X is optionally substituted with 1-3 groups of lower alkoxy, oxo or hydroxy.

X together with 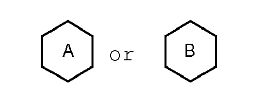 may also form 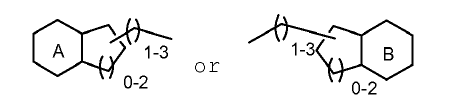 wherein one or two of carbon atoms constituting 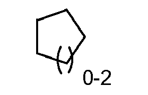 is optionally substituted with nitrogen, sulfur and oxygen; one or two double bonds is optionally contained in a ring; and one of carbon atoms constituting 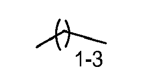 is optionally substituted with nitrogen, sulfur or oxygen.

Groups represented by 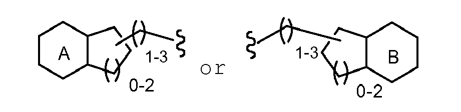 include, for example, groups represented by

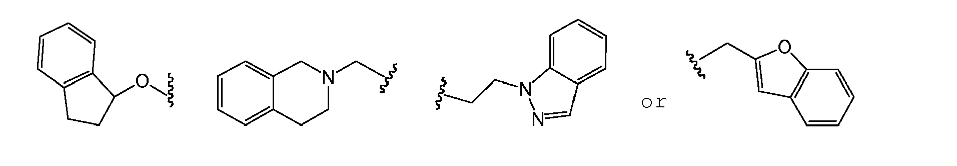

X is preferably lower alkylene having a main chain including three carbon atoms, wherein one or two of the carbon atoms constituting the main chain is optionally substituted with oxygen, sulfur or nitrogen, which lower alkylene is substituted with 1-3 same or different groups selected from the group consisting of lower alkyl, halogen and oxo; more preferably lower alkylene having a main chain including three carbon atoms, wherein one of the carbon atoms constituting the main chain is optionally substituted with oxygen, sulfur or nitrogen, which lower alkylene is substituted with one or two same or different groups selected from the group consisting of lower alkyl, halogen and oxo; further preferably a group selected from the group consisting of: 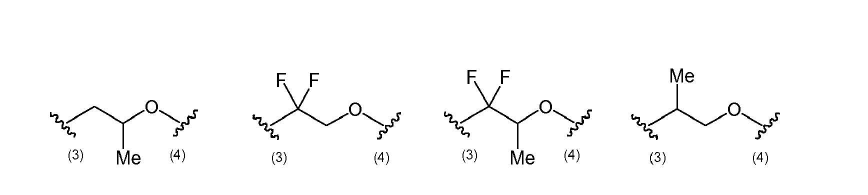 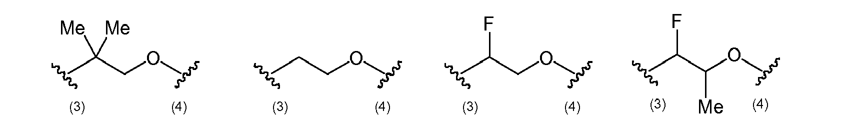 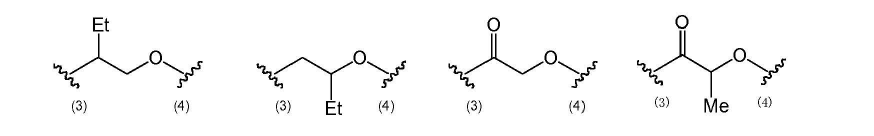 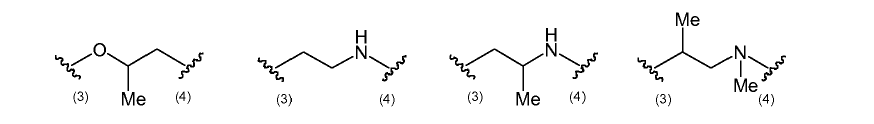 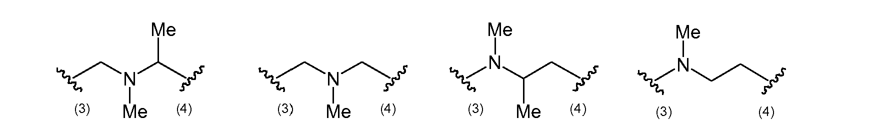 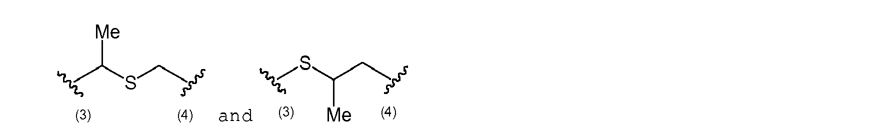 wherein 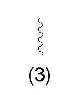 represents a binding site with 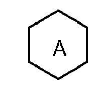 ; and 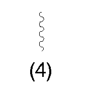 represents a binding site with

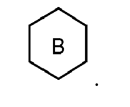

Y represents a group selected from the group consisting of hydrogen, lower alkyl optionally substituted with 1-3 same or different lower alkoxy groups or halogen atoms, lower alkoxy and halogen.

The lower alkyl represented by Y means an identical group as "lower alkyl" defined above. The lower alkyl is optionally substituted with 1-3 same or different lower alkoxy groups or halogen atoms.

The lower alkoxy represented by Y means an identical group as "lower alkoxy" defined above.

The term "halogen" represented by Y means an identical group as "halogen atom" defined above.

Y represents preferably hydrogen atom or lower alkyl optionally substituted with 1-3 same or different lower alkoxy groups or halogen atoms, more preferably hydrogen or methyl, further preferably hydrogen.

Any preferred embodiments of 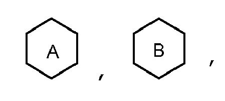 X and Y
described above may be combined.
A process for producing a compound according to an embodiment of the present invention will now be described.

A compound represented by a compound (I-A) according to an embodiment of the present invention can be produced, e.g., by the following process: 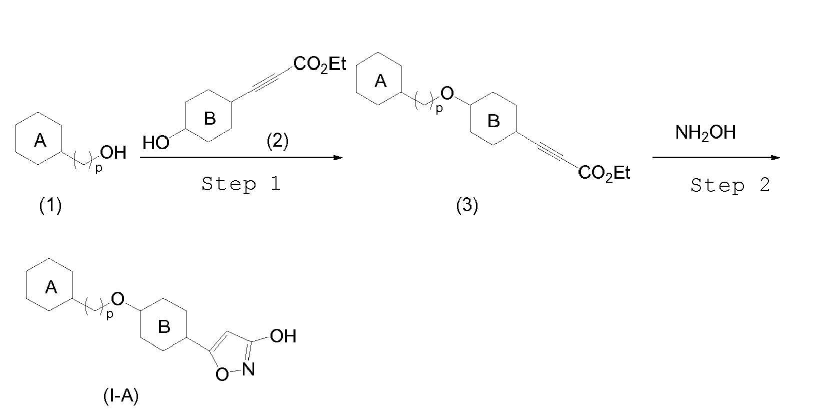 (wherein alkylene in 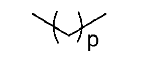 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings is optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino is optionally substituted with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups; and
   p represents an integer of from 1 to 3.

### (Step 1)

This step is a process of producing a compound (3) by reacting a compound (1) with a compound (2).

The reaction of the compound (1) with the compound (2) is a so-called Mitsunobu reaction, which may be performed by methods as described in documents (e.g., Mitsunobu, O., "The Use of Diethyl Azodicarboxylate and Triphenylphosphine in Synthesis and Transformation of Natural Products," Synthesis 1 (1981), pp. 1-28), methods equivalent thereto or combinations of these with usual methods in the presence of phosphine and azo compounds.

An amount of the compound (1) used in this step is typically 1-100 equivalents, preferably 1-5 equivalents, relative to 1 equivalent of the compound (2).

Examples of phosphine compounds used in this step include triphenylphosphine and triethylphosphine.

An amount of the phosphine compounds used is typically 1-100 equivalents, preferably 1-5 equivalents, relative to 1 equivalent of the compound (2).

Azo compounds used include, for example, ethyl azodicarboxylate and diisopropyl azodicarboxylate.

An amount of the azo compound used is typically 1-100 equivalents, preferably 1-5 equivalents, relative to 1 equivalent of the compound (2).

The reaction time in this step is typically 0.1-72 hours, preferably 0.5-24 hours.

The reaction temperature in this step is typically 0-200°C, preferably 0-50°C.

Reaction solvents used in this step include, but, unless interfering with the reaction, are not limited to, for example, tetrahydrofuran and diethyl ether.

The compound (3) thus obtained may be isolated and purified in well-known separation and purification method such as concentration, concentration under reduced pressure, crystallization, solvent extraction, reprecipitation and chromatography, or subjected to the next step without isolation or purification.

### (Step 2)

This step is a process of producing a compound (I-A) according to an embodiment of the present invention by reacting the compound (3) obtained in the step 1 with hydroxyamine in the presence of base.

Bases used in this step include, for example, sodium hydroxide and potassium hydroxide.

An amount of the base used is typically 1-100 equivalents, preferably 1-5 equivalents, relative to 1 equivalent of the compound (3).

An amount of hydroxyamine used is typically 1-100 equivalents, preferably 1-5 equivalents, relative to 1 equivalent of the compound (3).

The reaction time in this step is typically 0.1-72 hours, preferably 0.5-24 hours. The reaction temperature in this step is typically 0-100°C, preferably 0-40°C.

Reaction solvents used in this step include, but, unless interfering with the reaction, are not limited to, e.g., methanol and ethanol.

In Step 1, the compound (I-A) can be also produced by methods as described in Step 1, processes equivalent thereto or combinations of these with usual processes, using, instead of the compound (2), a compound represented by 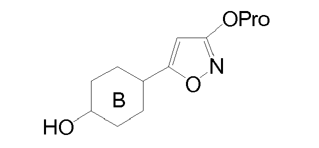 wherein Pro represents a protective group for hydroxy; and the other symbols have the same definitions specified above and then removing Pro.

The compound (I-A) according to an embodiment of the present invention thus obtained may be isolated and purified in well-known separation and purification method such as concentration, concentration under reduced pressure, crystallization, solvent extraction, reprecipitation and chromatography.

Furthermore, the compound (2) used may be commercially available or may be produced, for example, by a process illustrated below.

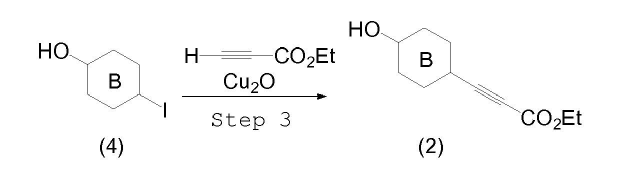

### (Step 3)

This step is a process of producing a compound (2) by reacting a compound (4) with ethyl propionate in the presence of copper (II) oxide.

An amount of ethyl propionate used in this step is typically 1-100 equivalents, preferably 1-5 equivalents, relative to 1 equivalent of the compound (4).

Examples of compounds (4) used in this step include, for example, 4-iodophenol, 4-iodophenyl methanol and 2-(4-iodophenyl)ethanol. Methyl propionate may be also used instead of ethyl propionate used in this step.

An amount of copper (II) oxide used in this step is typically 0.1-100 equivalents, preferably 1-5 equivalents, relative to 1 equivalent of the compound (4).

The reaction temperature in this step is typically 0-200°C, preferably 50-120°C.

The reaction time in this step is typically 0-72 hours, preferably 0.5-24 hours.

Reaction solvents used in this step include, but, unless interfering with the reaction, are not limited to, e.g., dimethylformamide and N-methylpyrrolidone.

The compound (2) thus obtained may be isolated and purified in well-known separation and purification measures such as concentration, concentration under reduced pressure, crystallization, solvent extraction, reprecipitation and chromatography, or subjected to the next step without isolation and purification.

The compound (3) may be also produced, for example, by the following process: 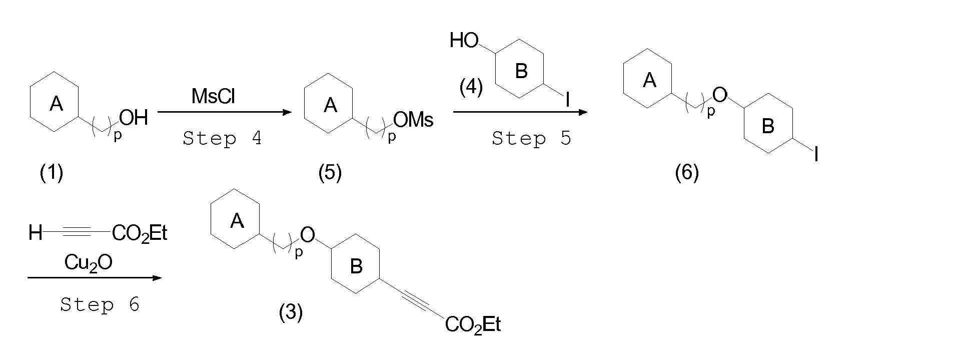 (wherein alkylene in 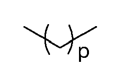 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings is optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino is optionally substituted with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups;
   p represents an integer of from 1 to 3; and
   the other symbols have the same definitions specified above).

### (Step 4)

This step is a process of producing a compound (5) by reacting the compound (1) with methanesulfonyl chloride (MsCl) in the presence of base.

Bases used in this step include, for example, triethylamine, diisopropylethylamine and pyridine.

An amount of the base used in this step is typically 0.1-100 equivalents, preferably 1-5 equivalents, relative to 1 equivalent of the compound (1).

An amount of methanesulfonyl chloride as used in this step is typically 1-100 equivalents, preferably 1-5 equivalents, relative to 1 equivalent of the compound (1).

The reaction time in this step is typically 0.1-24 hours, preferably 0.5-3 hours.

The reaction temperature in this step is typically 0-100°C, preferably 0-30°C.

Reaction solvents used in this step include, but, unless interfering with the reaction, are not limited to, e.g., ethyl acetate, chloroform and tetrahydrofuran.

The compound (5) thus obtained may be isolated and purified in well-known separation and purification method such as concentration, concentration under reduced pressure, crystallization, solvent extraction, reprecipitation and chromatography, or subjected to the next step without isolation and purification.

### (Step 5)

This step is a process of producing a compound (6) by reacting the compound (5) with the compound (4) in the presence of base.

Bases used in this step include, for example, sodium hydride and potassium carbonate.

An amount of the base used in this step is typically 1-100 equivalents, preferably 1-5 equivalents, relative to 1 equivalent of the compound (5).

An amount of the compound (4) used in this step is typically 1-100 equivalents, preferably 1-5 equivalents, relative to 1 equivalent of the compound (5).

The reaction temperature in this step is typically 0-200°C, preferably 0-100°C.

The reaction time in this step is typically 0.1-24 hours, preferably 0.5-5 hours.

Reaction solvents used in this step include, but, unless interfering with the reaction, are not limited to, e.g., dimethylformamide and N-methylpyrrolidone.

The compound (6) thus obtained may be isolated and purified in well-known separation and purification measures such as concentration, concentration under reduced pressure, reprecipitation, solvent extraction, crystallization and chromatography, or subjected to the next step without isolation and purification.

### (Step 6)

This step is a process of producing a compound (3) by reacting the compound (6) with ethyl propionate in the presence of copper (II) oxide.

The reaction in this step may be carried out by the methods as in Step 3, methods equivalent thereto or combinations of these with usual methods.

The compound (3) thus obtained may be isolated and purified in well-known separation and purification measures such as concentration, concentration under reduced pressure, reprecipitation, solvent extraction, crystallization and chromatography, or subjected to the next step without isolation and purification.

The compound (I-A) can be also produced by methods as described in Step 5, processes equivalent thereto or combinations of these with usual processes, using, instead of the compound (4) in Step 5, a compound represented by 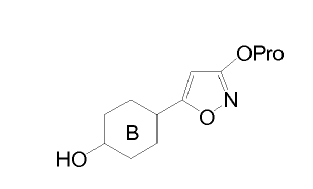 (wherein each symbol has the same definition specified above)
and then removing Pro.

In addition, a compound (I-B) according to an embodiment of the present invention can be produced, e.g., by the following process: 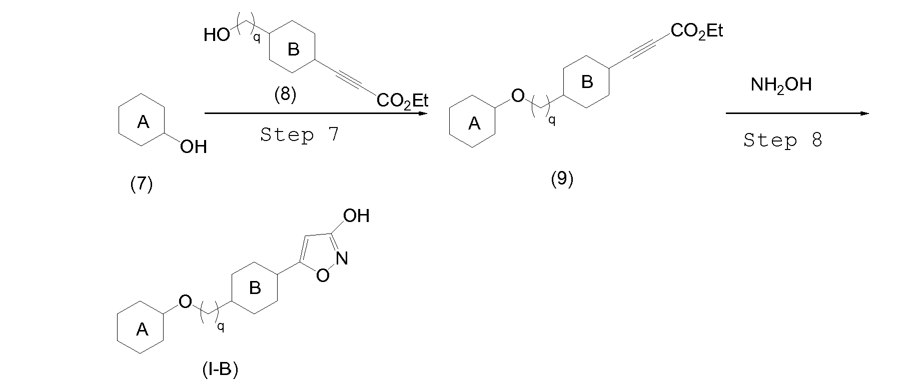 wherein alkylene in 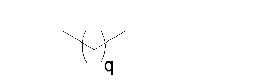 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings are optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino is optionally substituted with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups;
   q represents an integer of from 1 to 3; and
   the other symbols have the same definitions specified above.

### (Step 7)

This step is a process of producing a compound (9) by reacting a compound (7) with a compound (8). This step is a so-called Mitsunobu reaction, which may be performed by the methods as in the step 1, methods equivalent thereto or combinations of these with usual methods.

Compounds (8) used in this step include, for example, ethyl 3-(4-(hydroxymethyl)phenyl)-2-propinoate and ethyl (3-(4-(2-hydroxyethyl)phenyl)-2-propinoate.

The compound (9) thus obtained may be isolated and purified in well-known separation and purification method such as concentration, concentration under reduced pressure, reprecipitation, solvent extraction, crystallization and chromatography, or subjected to the next step without isolation and puritication.

### (Step 8)

This step is a process of producing a compound (I-B) according to an embodiment of the present invention by reacting the compound (3) obtained in the step 1 with hydroxyamine in the presence of base. The reaction in this step may be carried out by the methods as in the step 2, methods equivalent thereto or combinations of these with usual methods.

The compound (I-B) can be also produced by methods as described in Step 7, processes equivalent thereto or combinations of these with usual processes, using, instead of the compound (8) in Step 7, a compound represented by 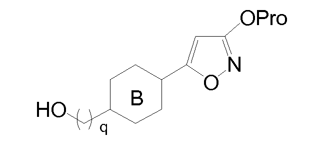 wherein each symbol has the same definition specified above and then removing Pro.

The compound (I-B) thus obtained may be isolated and purified in well-known separation and purification method such as concentration, concentration under reduced pressure, crystallization, solvent extraction, reprecipitation and chromatography.

In addition, a compound represented by a compound (I-C) according to an embodiment of the present invention: 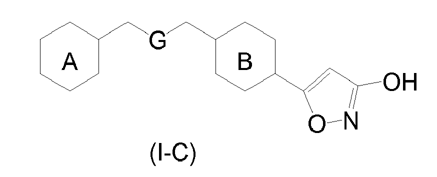 (wherein methylene in 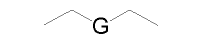 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings are optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino is optionally substituted with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups; and
   G represents NH, N-lower alkyl, sulfur or oxygen
   can be produced, e.g., by the following process: 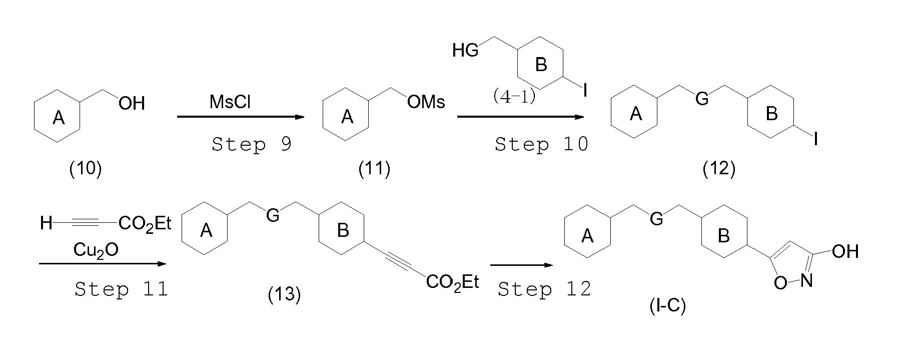 (wherein each symbol has the same definition specified above).

### (Step 9)

This step may be carried out by the methods as in the step 4, methods equivalent thereto or combinations of these with usual methods.

### (Step 10)

This step may be carried out by processes as in the step 5, processes equivalent thereto or combinations of these with usual processes.

### (Step 11)

This step may be carried out by processes as in the step 6, processes equivalent thereto or combinations of these with usual processes.

### (Step 12)

This step may be carried out by processes as in the step 2, processes equivalent thereto or combinations of these with usual processes.

The compound (I-C) can be also produced by methods as described in Step 10, processes equivalent thereto or combinations of these with usual processes, using a compound represented by 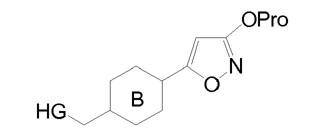 instead of a compound (4-1) in Step 10, and then removing Pro.

In addition, a compound represented by a compound (I-D) according to an embodiment of the present invention: 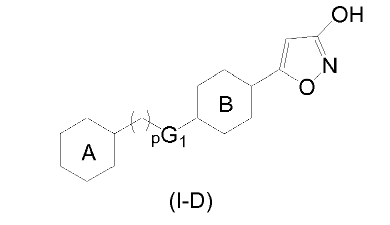 (wherein alkylene in 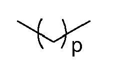 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings are optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino may is optionally with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups;
   G₁ represents NH, N-lower alkyl, sulfur; and
   the other symbols have the same definitions specified above)
   can be produced, e.g., by the following process: 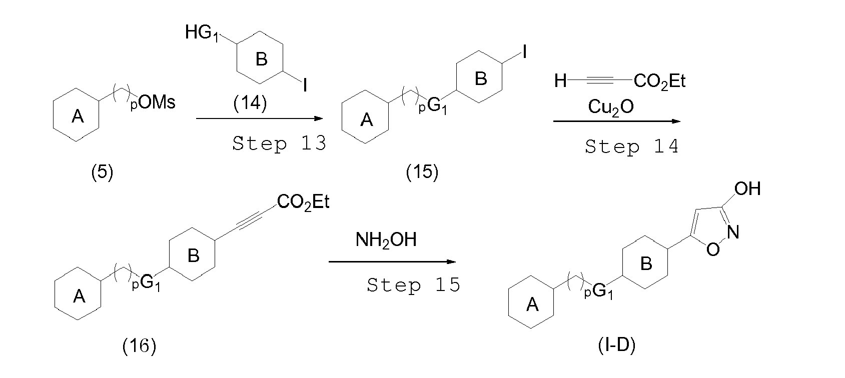 (wherein each symbol has the same definition specified above).

### (Step 13)

This step may be carried out by processes as in the step 5, processes equivalent thereto or combinations of these with usual processes.

### (Step 14)

This step may be carried out by processes as in the step 6, processes equivalent thereto or combinations of these with usual processes.

### (Step 15)

This step may be carried out by processes as in the step 2, processes equivalent thereto or combinations of these with usual processes.

The compound (I-D) can be also produced by methods as described in Step 13, processes equivalent thereto or combinations of these with usual processes, using, instead of a compound (14) in Step 13, a compound represented by 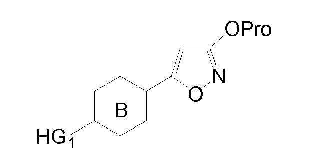 and then removing Pro.

In addition, a compound represented by a compound (I-E) according to an embodiment of the present invention: 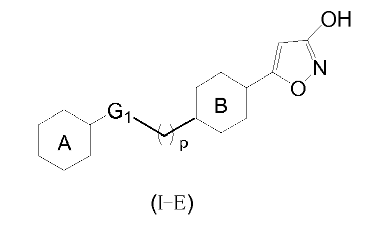 (wherein alkylene in 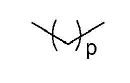 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings are optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino is optionally substituted with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups; and
   the other symbols have the same definitions specified above)
   can be produced, e.g., by the following process: 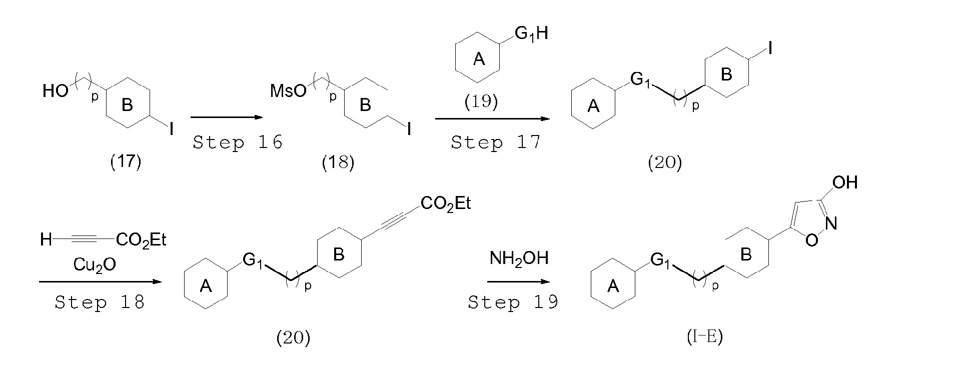 (wherein each symbol has the same definition specified above).

### (Step 16)

This step may be carried out by processes as in the step 4, processes equivalent thereto or combinations of these with usual processes.

### (Step 17)

This step may be carried out by processes as in the step 5, processes equivalent thereto or combinations of these with usual processes.

### (Step 18)

This step may be carried out by processes as in the step 6, processes equivalent thereto or combinations of these with usual processes.

### (Step 19)

This step may be carried out by processes as in the step 2, processes equivalent thereto or combinations of these with usual processes.

The compound (I-E) can be also produced by methods as described in Step 17, processes equivalent thereto or combinations of these with usual processes, using, instead of a compound (18) in Step 17, a compound represented by 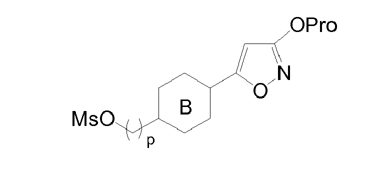 and then removing Pro.

In addition, a compound represented by a compound (I-F) according to an embodiment of the present invention: 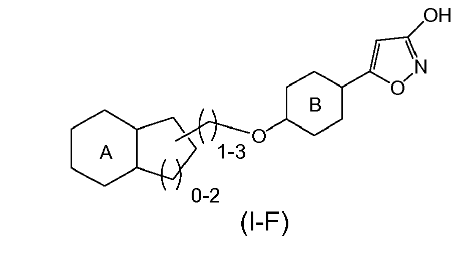 (wherein lower alkylene in 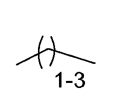 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings are optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino is optionally substituted with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups; and
   the other symbols have the same definitions specified above) can be produced, e.g., by the following process: 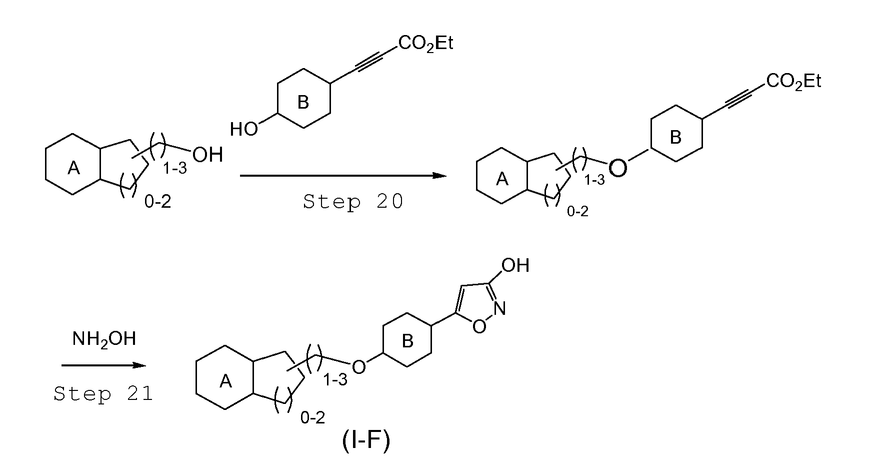 (wherein each symbol has the same definition specified above).

### (Step 20)

This step may be carried out by processes as in the step 1, processes equivalent thereto or combinations of these with usual processes.

### (Step 21)

This step may be carried out by processes as in the step 2, processes equivalent thereto or combinations of these with usual processes.

In addition, a compound represented by a compound (I-G) according to an embodiment of the present invention: 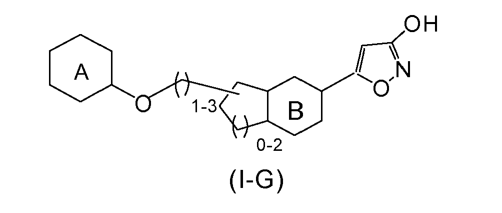 (wherein lower alkylene in 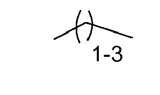 is optionally substituted with 1-3 same or different groups selected from the group consisting of
(1) amino optionally substituted with lower alkyl, lower alkylsulfonyl and lower alkanoyl,
(2) halogen,
(3) lower alkenyl,
(4) lower alkyl (when identical carbon atoms constituting the lower alkylene are substituted with two lower alkyl groups, the lower alkyl groups may together form 3- to 7-membered aliphatic rings, and one or two of carbon atoms constituting the aliphatic rings are optionally substituted with nitrogen, sulfur or oxygen),
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
   the lower alkyl, the lower alkynyl and the lower alkylidene are optionally substituted with 1-3 halogen atoms or hydroxy or lower alkoxy groups;
   the imino is optionally substituted with 1-3 lower alkyl, lower alkylsulfonyl, cyano, nitro and lower alkanoyl groups;
   the lower alkylene may have one or two double or triple bonds in a main chain and, in addition, the lower alkylene is optionally substituted with 1-3 lower alkoxy, oxo or hydroxy groups; and
   the other symbols have the same definitions specified above)
   can be produced, e.g., by the following process: 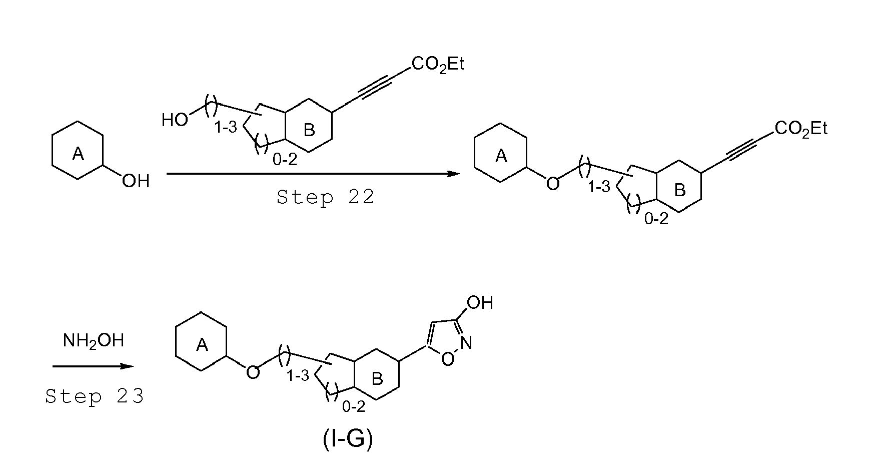 (wherein each symbol has the same definition specified above).

### (Step 22)

This step may be carried out by processes as in the step 1, processes equivalent thereto or combinations of these with usual processes.

### (Step 23)

This step may be carried out by processes as in the step 2, processes equivalent thereto or combinations of these with usual processes.

In addition, a compound represented by a compound (I-H) according to an embodiment of the present invention: 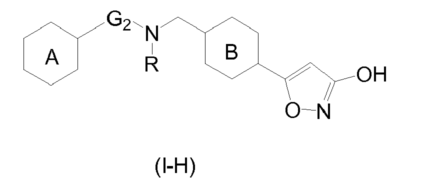 (wherein G₂ represents C(O) or S(O)₂;
R represents hydrogen or lower alkyl; and
the other symbols have the same definitions specified above) can be produced, e.g., by the following process: 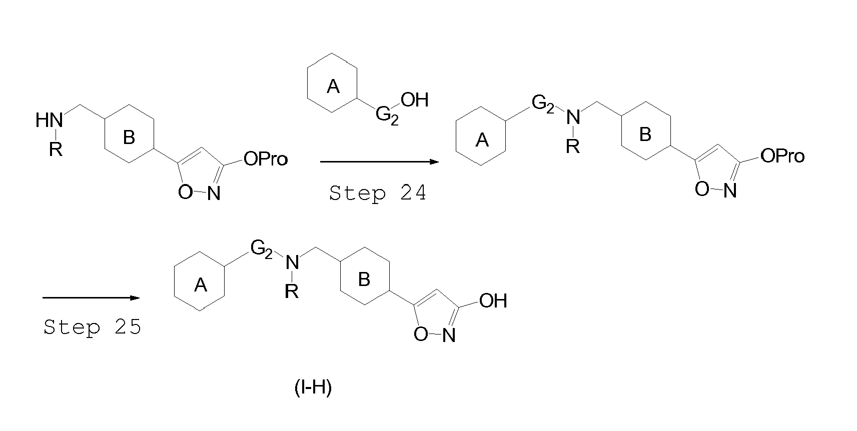 (wherein Pro represents a protective group for hydroxy; and the other symbols have the same definitions specified above).

### (Step 24)

This step is a process for producing an amide or sulfonamide compound by reacting an amino compound with a carboxylic acid or sulfonic acid compound. For this reaction, typical amide or sulfonamide formation reaction may be performed by methods as described in documents (e.g., Nobuo Izumiya, et al.: Peptide Gosei no Kiso to Jikken (Fundamentals and Experiments of Peptide Synthesis), Maruzen (1983); Comprehensive Organic Synthesis, Vol. 6, Pergamon Press (1991), etc.), methods equivalent thereto or combinations of these with usual methods, that is, by using a condensation agent that is well known to those skilled in the art, or by an ester activation method, a mixed anhydride method, an acid chloride method, a carbodiimide method, etc., which can be used by those skilled in the art. Examples of such amide formation reagents include thionyl chloride, oxalyl chloride, N,N-dicyclohexylcarbodiimide, 1-methyl-2-bromopyridinium iodide, N,N'-carbonyldiimidazole, diphenylphosphoryl chloride, diphenylphosphoryl azide, N,N'-disuccinimidyl carbonate, N,N'-disuccinimidyl oxalate, 1-ethyl-3-(3-dimethylaminopropyl)carbodiimide hydrochloride, ethyl chloroformate, isobutyl chloroformate and benzotriazol-1-yl-oxy-tris(dimethylamino)phosphonium hexafluorophosphate; especially preferably, e.g., thionyl chloride, 1-ethyl-3-(3-dimethylaminopropyl)carbodiimide hydrochloride, N,N-dicyclohexylcarbodiimide and
benzotriazol-1-yl-oxy-tris(dimethylamino)phosphonium hexafluorophosphate. For the amide or sulfonamide formation reaction, base and a condensation adjuvant may be also used together with the amide or sulfonamide formation reagent.

Bases as used include ternary aliphatic amines such as trimethylamine, triethylamine, N,N-diisopropylethylamine, N-methylmorpholine, N-methylpyrrolidine, N-methylpiperidine, N,N-dimethylaniline, 1,8-diazabicyclo[5.4.0]undeca-7-ene (DBU) and 1,5-azabicyclo[4.3.0]nona-5-ene (DBN); and aromatic amines such as pyridine, 4-dimethylaminopyridine, picoline, lutidine, quinoline and isoquinoline; especially preferably, e.g., ternary aliphatic amines, etc., particularly preferably, e.g., triethylamine, N,N-diisopropylethylamine, etc.

Condensation aid as used include, for example, N-hydroxybenzotriazole hydrate, N-hydroxysuccinimide, N-hydroxy-5-norbornen-2,3-dicarboximide or 3-hydroxy-3,4-dihydro-4-oxo-1,2,3-benzotriazole; especially preferably, e.g., N-hydroxybenzotriazole, etc.

An amount of amino compound as used is typically 0.1-10 equivalents, preferably 0.5-3 equivalents, relative to 1 equivalent of carboxylic or sulfonic acid or a reactive derivative thereof, depending on the types of compound and solvent used and other reaction conditions.

An amount of amide formation reagent as used is typically 1-10 equivalents, preferably 1-3 equivalents, relative to 1 equivalent of carboxylic or sulfonic acid or a reactive derivative thereof, depending on the types of compound and solvent used and other reaction conditions.

An amount of condensation adjuvant as used is typically 1-10 equivalents, preferably 1-3 equivalents, relative to 1 equivalent of carboxylic or sulfonic acid or a reactive derivative thereof, depending on the types of compound and solvent used and other reaction conditions.

An amount of base as used is typically 1-10 equivalents, preferably 1-5 equivalents, per equivalent of carboxylic or sulfonic acid or a reactive derivative thereof, depending on the types of compound and solvent used and other reaction conditions.

Unless interfering with the reaction, reaction solvents as used in this step, which are not particularly limited, include, e.g., inactive solvents; specifically, e.g., methylene chloride, chloroform, 1,2-dichloroethane, N,N-dimethylformamide, ethyl acetate, methyl acetate, acetonitrile, benzene, xylene, toluene, 1,4-dioxane, tetrahydrofuran and dimethoxyethane or mixed solvents thereof; preferably, e.g., methylene chloride, chloroform, 1,2-dichloroethane, acetonitrile or N,N-dimethylformamide, from the viewpoint of ensuring preferable reaction temperature.

The reaction temperature is typically from -78°C to the boiling point of a solvent, preferably 0-30°C.

The reaction time is typically 0.5-96 hours, preferably 3-24 hours.

One or a combination of two or more of bases, amide or sulfonamide formation reagents and condensation adjuvants as used in this step may be used.

The amide or sulfonamide compounds thus obtained may be isolated and purified in well-known separation and purification measures such as concentration, concentration under reduced pressure, reprecipitation, solvent extraction, crystallization and chromatography, or subjected to the next step without isolation and purification.

### (Step 25)

This step is a process for producing a compound (I-H) by removing a protective group for hydroxy of isoxazole.

A reaction in this step can be carried out by a method as described in the document (T.W. Green: Protective Groups in Organic Synthesis, Second Edition, John Wiley & Sons (1991)), methods equivalent thereto or combinations of these with usual methods. Protective groups for hydroxy include, for example, MOM (methoxymethoxy).

In case of production of a compound (I) according to an embodiment of the present invention or a compound represented by the formula (I-A), (I-B), (I-C), (I-D), (I-E), (I-F), (I-G) or (I-H) encompassed by the formula (I), when X or 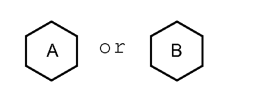 has substituents requiring a protective group, a compound according to an embodiment of the present invention can be produced by optionally introducing an appropriate protective into the substituents and then deprotecting the substituents.

Although Pro means a protective group for hydroxy group, an appropriately needed protective group may be used in each step. Pro includes, for example, MOM (methoxymethoxy).

The protective group can be introduced and removed by a method as described in the document (T.W. Green: Protective Groups in Organic Synthesis, Second Edition, John Wiley & Sons (1991)), methods equivalent thereto or combinations of these with usual methods.

Compounds in accordance with embodiments of the present invention may be present as pharmaceutically acceptable salts, which can be produced according to usual methods using the compound represented by the formula (I), and (I-A), (I-B), (I-C), (I-D), (I-E), (I-F), (I-G) or (I-H) encompassed thereby.

The acid-addition salts include, for example, hydrohalides such as hydrochlorides, hydrofluorides, hydrobromides, hydroiodides; inorganic acid salts such as nitrates, perchlorates, sulfates, phosphates, carbonates; lower alkylsulfonates such as methanesulfonates, trifluoromethanesulfonates, ethanesulfonates; arylsulfonates such as benzenesulfonates, p-toluenesulfonates; organic acid salts such as fumarates, succinates, citrates, tartrates, oxalates, maleates; other organic acid-addition salts with amino acid such as glutamates, aspartates.

When the compounds of the invention have an acid group in the molecule, for example, when they have a carboxyl group, then the compounds may be processed with a base so as to convert them into the corresponding pharmaceutically-acceptable salts. The base-addition salts include, for example, alkali metal salts with sodium or potassium; alkaline earth metal salts with calcium or magnesium; ammonium salts; organic base-addition salts with guanidine, triethylamine, dicyclohexylamine, etc

Furthermore, the compounds of the invention may also be in any other form of hydrates or solvates of their free compounds or their salts.

In contrast, a salt or ester can be also converted into a free compound by an ordinary method.

Depending on the type of the substituents therein, the compounds of the invention include stereoisomers and tautomers such as optical isomers, diastereomeric isomers and geometrical isomers. Needless-to-say, the compounds of the invention include all these isomers. Further needless-to-say, the compounds of the invention include all mixtures of such isomers.

In producing medicines for prevention and remedy for type II diabetes or diseases or symptoms associated with it, the compounds of formula (I) of the invention may be combined with carrier.

The dose of the compounds of formula (I) of the invention for prevention or remedy for diseases naturally varies, depending on the property of the symptom to which the treatment is directed, the specific compound selected for it and the administration route.

The dose also varies depending on the age, the body weight and the sensitivity of patients.

In general, the daily dose for one-time or plural-times administration may be from about 0.001 mg/kg-body weight to about 100 mg/kg-body weight, preferably from about 0.01 mg/kg-body weight to about 50 mg/kg-body weight, even more preferably from about 0.1 mg/kg-body weight to about 10 mg/kg-body weight. As the case may be, administration of a dose over the range may be necessary.

An example of a suitable dose for oral administration is described. The daily dose for one-time or two- to four-times administration may be at least from about 0.01 mg to at most 2.0 g. Preferably, the daily administration frequency is once or twice a day, and the daily dose is from about 1.0 mg to about 200 mg. More preferably, the daily dose is from about 10 mg to 100 mg for one-time administration a day.

For intravenous administration or oral administration, a typical dose of the compound (I) may be from about 0.001 mg/day/kg-body weight to about 100 mg/day/kg-body weight (preferably from 0.01 mg/day/kg-body weight to about 10 mg/day/kg-body weight), more preferably from about 0.1 mg/day/kg-body weight to 10 mg/day/kg-body weight.

As so mentioned hereinabove, the pharmaceutical composition of the invention comprises a compound of formula (I) and a pharmaceutically-acceptable carrier. The term "composition" is meant to contain not only a product produced by directly or indirectly combining, hybridizing or aggregating 2 or more ingredients, a product produced as a result of dissociation of one or more ingredients, or a compound produced as a result of reaction or interaction of different types of ingredients, but also an active and inactive ingredient of constituting a carrier (pharmaceutically-acceptable vehicle).

As combined with a pharmaceutically-acceptable carrier, the composition of the invention preferably contains a compound of formula (I) in an amount effective for remedy and prevention of type II diabetes and for retardation of the onset of the disease.

For administering the effective dose of the compound of the invention to mammals, especially to humans, employable is any suitable administration route. For example, the route may be oral administration, rectal administration, local administration, intravenous administration, ophthalmic administration, lung administration or nasal administration. Examples of the administration forms are tablets, troches, powders, suspensions, solutions, capsules, creams, aerosols. Preferred are oral tablets.

In preparing oral compositions, usable are any ordinary pharmaceutical media. Their examples are water, glycol, oil, alcohol, fragrant additives, preservatives, colorants. In preparing liquid compositions for oral administration, for example, mentioned are suspensions, elixirs and solutions. Their carriers are, for example, starch, sugar, microcrystalline cellulose, diluent, granulating promoter, lubricant, binder, disintegrator. In preparing solid compositions for oral administration, for example, mentioned are powders, capsules and tablets. Above all, such solid compositions for oral administration are preferred.

In view of the easiness in their administration, tablets and capsules are the most advantageous forms for oral administration. If desired, the tablets may be coated according to standard aqueous or non-aqueous coating techniques.

In addition to the above-mentioned ordinary administration modes for them, the compounds of formula (I) may also be administered according to controlled release systems and/or controlled delivery systems, for example, as in US Patents 3,845,770, 3,916,899, 3,536,809, 3,598,123, 3,630,200 and 4,008,719.

The pharmaceutical composition of the invention suitable for oral administration includes capsules, cashews and tablets that contain a predetermined amount of the active ingredient in the form of powders or granules thereof, or in the form of water-soluble liquids, water-insoluble liquids, oil-in-water emulsions or water-in-oil emulsions thereof. These compositions may be prepared in any pharmaceutical methods, and all the methods include a process of combining the active ingredient with a carrier of one or more necessary ingredients.

In general, the active ingredient is uniformly and fully mixed with a liquid carrier, or a well-separated solid carrier or with both the two, and then, if desired, the product is shaped into suitable forms to prepare the composition. For example, tablets are produced through compression and shaping, optionally along with one or more side components. Using a suitable machine, compressed tablets may be produced by mixing the active ingredient optionally with binder, lubricant, inert vehicle, surfactant or dispersant and compressing the resulting mix in any desired manner into powders or granules.

Shaped tablets may be prepared by shaping a mixture of a powdery wet compound and an inert liquid diluent, using a suitable machine.

Preferably, the tablets each contain from about 1 mg to 1 g of the active ingredient; and the cashews and the capsules each contain from about 1 mg to 500 mg of the active ingredient.

Examples of the administration modes of the compounds of formula (I) for pharmaceutical use are as follows:

**Table 1**

| Suspension for Injection (I. M.) | |
|---|---|
| | mg/ml |
| compound of formula (I) | 10 |
| methyl cellulose | 5,0 |
| Twen 80 | 0,5 |
| beszyl alcohol | 9,0 |
| benzalkonium chloride | 1,0 |
| water for injection added to make 1.0 ml | |

**Table 2**

| Tablets | mg/tablet |
|---|---|
| compound of formula (I) | 25 |
| methyl cellulose | 415 |
| Tween 80 | 14.0 |
| benzyl alcohol | 43.5 |
| magnesium stearate | 2,5 |
| total | 500 mg |

**Table 3**

| Capsules | mr/capsule |
|---|---|
| compound of formula (I) | 25 |
| lactose powder | 573.5 |
| magnesium stearate | 1.5 |
| total | 600 mg |

**Table 4**

| Aerosol | per one container |
|---|---|
| compound of formula (I) | 24 mg |
| lecithin NF Liq. Conc. | 1.2 mg |
| trichlorofluoromethane, NF | 4.025 g |
| dichlorofluoromethane, NF | 12.15 g |

The compounds of formula (I) may be used, as combined with any other drugs usable not only for type II diabetes-associated diseases or symptoms but also for remedy/prevention/retardation of the onset of type II diabetes. The additional drugs may be administered in any administration route and dose generally employed in the art, simultaneously with or separately from the compound of formula (I).

In case where the compound of formula (I) is used along with one or more other drugs, then a pharmaceutical composition comprising the compound of formula (I) and the additional drug is preferred. Accordingly, the pharmaceutical composition of the invention may comprise not only the compound of formula (I) but also one or more such active ingredients. Examples of the active ingredients that may be combined with the compounds of formula (I) are mentioned below, which, however, are not limitative. These may be separately administered or may be administered simultaneously as contained in the same pharmaceutical composition.
(a) other GPR120 agonist,
(b) glucokinase activators,
(c) bis-guanides (e.g., buformin, metoformin, fenformin,),
(d) PPAR agonists (e.g., triglytazon, pioglytazon, rosiglytazon),
(e) insulin,
(f) somatostatin,
(g) α-glucosidase inhibitors (e.g., boglybose, miglytol, acarbose),
(h) insulin secretion promoters (e.g., acetohexamide, calbutamide, chlorpropamide, glybomlide, glycrazide, glymerpiride, glypidide, glyquidine, glysoxepide, glyburide, glyhexamide, glypinamide, fenbutamide, trazamide, tolbutamide, tolcyclamide, nateglynide, repaglynide),

(i) DPP-IV (dipeptidyl peptidase IV) inhibitors, and

The weight ratio of the compound of formula (I) to the second active ingredient may vary within a broad range, and depends on the effective amount of the individual active ingredients. Accordingly, for example, when the compound of formula (I) is combined with a PPAR agonist, then the weight ratio of the compound of formula (I) to the PPAR agonist may be generally from about 1000/1 to 1/1000, preferably from about 200/1 to 1/200. The combination of the compound of formula (I) and the other active ingredient may be within the above-mentioned range. In any case, an effective amount of the individual ingredients should be in the combination.

The compound according to an embodiment of the present invention has a GPR120 function regulating action, wherein "GPR120 function regulating action" means activation or suppression of the function of a GPR120 receptor. For example, a GPR120 agonist is also included in compounds having the GPR120 function regulating action.

A compound according to an embodiment of the present invention or a pharmaceutically acceptable salt thereof has a GPR120 function regulating action, particularly a GPR120 agonist action, and is useful for treating and/or preventing diabetes mellitus or hyperlipidemia.

It should be understood by those skilled in the art that various modifications, combinations, sub-combinations and alterations may occur depending on design requirements and other factors insofar as they are within the scope of the appended claims or the equivalents thereof.

### EXAMPLES

The present invention is described below in more detail referring to Formulation Examples, Examples and Reference Examples, but is not limited thereto.

### Formulation Example 1

Ten parts of the compound in accordance with Example 1,15 parts of heavy magnesium oxide and 75 parts of lactose are blended uniformly to prepare a powder having a particle size of 350 µm or less in powder or granular form. The powder is charged in a capsule container to form a capsule.

### Formulation Example 2

After uniformly blending 45 parts of the compound in accordance with Example 1,15 parts of starch, 16 parts of lactose, 21 parts of crystalline cellulose, 3 parts of polyvinylalcohol and 30 parts of distilled water, the blend is crushed into granules, which are dried and then sieved to form granules having a particle diameter of 177-1410 µm.

### Formulation Example 3

After preparing granules in the same manner as in Formulation Example 2, 3 parts of calcium stearate is added to 96 parts of the granules, and the mixture is compression-molded to prepare tablets having a diameter of 10 mm.

### Formulation Example 4

To 90 parts of the granules prepared by the method described in Formulation Example 2 is added 10 parts of crystalline cellulose and 3 parts of calcium stearate, and the mixture was compression-molded to form tablets having a diameter of 8 mm, to which a syrup gelatin/precipitated calcium carbonate suspension is added to prepare sugar-coated tablets.

Wakogel (registered trademark) C-300, made by Wako Pure Chemical Industries Ltd., or KP-Sil (Registered Trademark) Silica prepacked column, made by Biotage, was used for the silica gel column chromatography in Examples. Kieselgel^{™} 60 F₂₅₄, Art. 5744, made by Merck & Co., was used for preparative thin layer chromatography. Chromatorex (registered trademark) NH (100-250 mesh or 200-350 mesh), made by Fuji Silysia Chemical Ltd., was used for basic silica gel column chromatography.

¹H-NMR was measured using Gemini (200 MHz, 300 MHz), Mercury (400 MHz) and Inova (400 MHz), made by Varian, using tetramethylsilane as a standard substance. In addition, the mass spectra were measured by electrospray ionization (ESI) or atmospheric pressure chemical ionization (APCI) using Micromass ZQ made by Waters.

The meanings of the abbreviations in Examples are shown below.
i-Bu = isobutyl
n-Bu = n-butyl
t-Bu = tert-butyl
Boc = tert-butoxycarbonyl
Me = methyl
Et = ethyl
Ph = phenyl
i-Pr = isopropyl
n-Pr = n-propyl
CDCl₃ = heavy chloroform
CD₃OD = heavy methanol
DMSO-d₆ = heavy dimethylsulfoxide

The meanings of the abbreviations in the nuclear magnetic resonance spectra are shown below.
s = singlet
d = doublet
dd = double doublet
dt = double triplet
ddd = double double doublet
Sept = septet
t = triplet
m = multiplet
br = broad
brs = broad singlet
q = quartet
J = coupling constant
Hz = hertz

### Example 1

### 5-(4-(2-(3-isopropoxyphenyl)-1-methylethoxy)phenyl)isoxazol-3-ol

### 1) Production of methyl(3-hydroxyphenyl)acetate

To a solution of3-hydroxyphenylacetic acid (2.0 g) in methanol (10 ml), 250 mg of tosic acid monohydrate and 2.9 ml oftrimethoxymethane were added, and the reaction solution was heated to reflux for 5 hours. The reaction solution was cooled, and thereafter the solvent was removed under reduced pressure. The residual residue was diluted with diethyl ether, washed with a saturated aqueous sodium bicarbonate solution, and dried with anhydrous magnesium sulfate. The solvent was removed under reduced pressure, and the resultant residue was purified by silica gel column chromatography (eluent: ethyl acetate/hexane (0:100-50:50)) to yield the title compound as a colorless oil.

### 2) Production of methyl(3-isopropoxyphenyl)acetate

To a solution of methyl(3-hydroxyphenyl)acetate (200 mg) in tetrahydrofuran (5 ml), 0.15 ml of isopropyl alcohol, 480 mg of triphenylphosphine, and 0.82 ml of diethyl azodicarboxylate (45% toluene solution) were added, and the reaction solution was stirred overnight at room temperature. The reaction solvent was removed under reduced pressure, and the resultant residue was purified by silica gel column chromatography (eluent: ethyl acetate/hexane (0:100-25:75-60:40)) to yield the title compound as a colorless oil.

### 3) Production of 2-(3-isopropoxyphenyl)ethanol

To a solution of methyl(3-isopropoxyphenyl)acetate (280 mg) in tetrahedron (5 ml), 52 mg of lithium aluminum hydride was added under ice-cooling, and the reaction solution was stirred at the same temperature for 25 minutes. To the reaction solution was added sodium sulfate decahydrate, and the mixture was stirred at room temperature for 2 hours. The reaction liquid was filtered, and thereafter the filtrate was removed under reduced pressure. The resultant residue was purified by silica gel column chromatography (eluent: ethyl acetate/hexane (0:100-50:50) to yield the title compound as a colorless oil.

### 4) Production of (3-isopropoxyphenyl)acetaldehyde

To a solution of 2-(3-isopropoxyphenyl)ethanol (200 mg) in chloroform (7 ml), 700 mg of Dess-Martin reagent was added, and the reaction solution was stirred at room temperature for 2 hours. Furthermore, 280 mg of Dess-Martin reagent was added to the reaction solution, and the mixture was stirred at room temperature for 1 hour. The reaction solution was filtered through Celite, and the filtrate was diluted with a saturated aqueous sodium bicarbonate solution, extracted with chloroform and dried over anhydrous magnesium sulfate. The solvent was removed under reduced pressure, and the resultant residue was purified by silica gel column chromatography (eluent: ethyl acetate/hexane (0:100-30:70) to yield the title compound as a yellow oil.

### 5) Production of 1-(3-isopropoxyphenyl)-2-propanol

To a solution of (3-isopropoxyphenyl)acetaldehyde (116.6 mg) in tetrahydrofuran (1 ml), 1.0 ml of methyllithium was added under ice-cooling, and the reaction solution was stirred at the same temperature for 1 hour. The reaction solution was diluted with a saturated aqueous ammonium chloride solution, extracted with ethyl acetate and dried over anhydrous magnesium sulfate. The solvent was removed under reduced pressure, and the residual residue was purified by preparative thin layer chromatography (Kieselgel^{™} 60 F₂₅₄, Art. 5744, made by Merck & Co.; ethyl acetate/hexane (30:70)) to yield the title compound as a colorless oil.

### 6) Production of 5-(4-(2-(3-isopropoxyphenyl)-1-methylethoxy)phenyl)isoxazol-3-ol

To a solution of 1-(3-isopropoxyphenyl)-2propanol (87 mg) in tetrahydrofuran (2 ml), ethyl 3-(4-hydroxyphenyl)-2-propinoate (103 mg) obtained in Reference Example 1, 142 mg of triphenylphosphine, 0.245 ml of azodicarboxylic acid diethyl ester (2.2M toluene solution) were added under ice-cooling, and the reaction solution was stirred at room temperature for 1 hour. After addition of excess of methanol, the solvent was removed under reduced pressure and the resultant residue was purified by silica gel column chromatography (eluent: hexane/ethyl acetate (98:2-50:50) to yield the title compound as a colorless oil. To a crude product obtained in methanol (1ml), 69 mg of hydroxylamine hydrochloride and 0.3 ml of 5N aqueous potassium hydroxide solution were added, and the reaction solution was stirred overnight at room temperature. A 10% aqueous citric acid solution was added to the reaction solution, and the mixture was extracted with chloroform. The organic layer was dried over anhydrous sodium sulfate. then the solvent was removed under reduced pressure, and the resultant residue was purified through reversed phase medium pressure liquid chromatography (ODS-AS-360-CC (made by YMC), mobile phase: water-acetonitrile-0.1% trifluoroacetic acid) The solvent was removed under reduced pressure, the resultant residue was diluted with chloroform and washed with a saturated saline solution, and the organic layer was dried over anhydrous sodium sulfate. The solvent was removed under reduced pressure to yield the title compound as a pale yellow oil.
¹HNMR(400MHz,CDCl₃)δ:1.29-1.35(9H,m),2.80(1H,dd,J=13.7,6.7Hz),3.07(1H,dd,J= 13.7,5.9Hz),4.47-4.58(1H,m),4.59-4.70(1H,m),6.07(1H,s),6.71-6.84(3H,m),6.94(2H,d,J =8.8Hz),7.19(1H,t,J=7.7Hz),7.63(2H,d,J=8.8Hz)
ESI-MS Found:m/z 354[M+H]+

### Example 2

### 5-(4-(2-fluoro-2-(3-methoxyphenyl)ethoxy)phenyl)isoxazol-3-ol

### 1) Production of 2-(4-iodophenoxy)-1-(3-methoxyphenyl)ethanone

To a solution of 2-bromo-1-(3-methoxyphenyl)ethanone (1.0 g) in dimethylformamide (10 ml), 1.44 g of 4-iodophenol and 904 mg of potassium carbonate were added, and the reaction solution was stirred overnight at 80°C. The reaction solution was cooled, 1N hydrochloric acid was then added, and the mixture was extracted with ethyl acetate. The combined organic layers were washed with saturated saline solution and dried over anhydrous sodium sulfate. The solvent was removed under reduced pressure, and the resultant residue was purified by silica gel column chromatography (eluent: hexane/ethyl acetate (98:2-50:50)) to yield the title compound as a yellow oil.

### 2) Production of 1-(1-fluoro-2-(4-iodophenoxy)ethyl)-3-methoxybenzene

To a solution of 2-(4-iodophenoxy)-1-(3-methoxyphenyl)ethanone (448 mg) in methanol (5 ml), 92 mg of sodium borohydride was added under ice-cooling, and the reaction solution was stirred at room temperature for 30 minutes. A 10% aqueous citric acid solution was added to the reaction solution, and the mixture was extracted with ethyl acetate. The combined organic layers were washed with saturated saline solution and then dried over anhydrous sodium sulfate. The solvent was removed under reduced pressure to yield a crude product as a colorless oil. To a solution of the crude product in chloroform (3 ml), 0.45 ml of deoxo-fluoro(bis(2-methoxyethyl)aminosulfur trifluoride) was added under ice-cooling, and the reaction solution was stirred at room temperature for 1 hour. The solvent was removed under reduced pressure, and the resultant residue was purified by silica gel column chromatography (eluent: hexane/ethyl acetate (98:2-50:50) to yield the title compound as a colorless oil.

### 3) Production of 5-(4-(2-fluoro-2-(3-methoxyphenyl)ethoxy)phenyl)isoxazol-3-ol

To a solution of 1-(1-fluoro-2-(4-iodophenoxy)ethyl)-3-methoxybenzene (0.22 g) in dimethylformamide (3 ml), 0.18 ml of ethyl propiolate and 0.08 g of copper (II) oxide were added, and the reaction solution was stirred at 110°C for 15 hours. The reaction solution was cooled, then Celite-filtered and the filtrate was removed under reduced pressure. The resultant residue was dissolved in 1 ml of ethanol, 0.05ml of 50% aqueous hydroxylamine solution and 0.03ml of 2N sodium hydroxide solution were added, and the reaction solution was stirred overnight at room temperature. The reaction solution was acidified with 10% of citric acid solution, and extracted with chloroform, and dried over anhydrous magnesium sulfate. The solvent was removed under reduced pressure, the resultant residue was purified by preparative thin layer chromatography (Kieselgel^{™} 60 F₂₅₄, Art5744, made by Merck & Co., chloroform/methanol= 8:1) to yield the title compound as a light brown solid.
¹H-NMR(400MHz,CDCl₃)δ:3.84(3H,s),4.19-4.26(1H,m),4.31-4.40(1H,m),5.82(1H,dd, J=48.0,5.8Hz),6.10(1H,s),6.94(1H,d,J=8.4Hz),6.92-7.00(4H,m),7.35(1H,t,J=7.7Hz),7.6 7(2H,d,J=8.6Hz)
ESI-MS Found:m/z 330[M+H]+

### Example 3

### 5-(4-(2,2-difluoro-2-(3-methoxyphenyl)ethoxy)phenyl)isoxazol-3-ol

### 1) Production of 1-(1,1-difluoro-2-(4-iodophenoxy)ethnyl)-3-methoxybenzene

To a solution of 2-(4-iodophenoxy)-1-(3-methoxyphenyl)ethanone (393 mg) in chloroform (2 ml), 0.394 ml of bis(2-methoxyethyl)aminosulfur trifluoride was added, and the reaction solution was stirred at 50°C for 24 hours. To the reaction solution was added 0.013 ml of ethanol, and the mixture was further stirred at the same temperature for 24 hours. The reaction solution was cooled, the solvent was then removed, and the resultant residue was purified by silica gel column chromatography (eluent: hexane/ethyl acetate (98:2-50:50) to yield the title compound as a brown oil.

### 2) Production of 5-(4-(2,2-difluoro-2-(3-methoxyphenyl)ethoxy)phenyl)isoxazol-3-ol

The title compound was obtained as a white solid by the method as in Example 2, methods equivalent thereto or combinations of these with usual methods, using 1-(1,1-difluoro-2-(4-iodophenoxy)ethyl)-3-methoxybenzene.
¹HNMR(400MHz,CDCl₃)δ:3.86(3H,s),4.41(2H,t,J=11.9Hz),6.11(1H,s),6.97(2H,d,J=8. 4Hz),7.02(1H,d,J=7.8Hz),7.12(1H,s),7.17(1H,d,J=7.8Hz),7.39(1H,t,J=7.8Hz),7.66(2H,d ,J=8.4Hz)
ESI-MS Found:m/z 348[M+H]+

### Example 4

### 5-(4-(2-(3-isopropoxyphenyl)propoxy)phenyl)-isoxazol-3-ol

In tetrahydrofuran, 100 mg of 4-(3-(methoxymethoxy)isoxazol-5-yl)phenol and 88 mg of 2-(3-isopropoxyphenyl)propan-1-ol were dissolved, 237 mg of triphenylphosphine and 183 mg of diisopropyl azodicarboxylate were added, and the mixture was stirred at room temperature. Methanol was added to the reaction liquid, the solvent was removed, and the resultant residue was purified by silica gel chromatography. To the product was added 1N hydrochloric acid/methanol, and the mixture was stirred at room temperature and was concentrated after confirmation of deprotection of an MOM group. The title compound was obtained by purifying the residue by silica gel chromatography.
¹H-NMR(400MHz,CDCl₃)δ:7.64(2H,d,J=8.8Hz),7.26-7.22(1H,m),6.94(2H,d,J=8.8Hz), 6.87-6.81(2H,m),6.78(1H,dd,J=8.2,2.2Hz),6.07(1H,s),4.56(1H,sept,J=6.1Hz),4.12(1H,d d,J=9.0,6.4Hz),3.99(1H,dd,J=9.0,7.0Hz),3.22(1H,qdd,J=6.4,6.4,7.0Hz),1.41(3H,d,J=6.4 Hz),1.34 (6H,d,J=6.1Hz).
ESI-MS Found:m/z 354[M+H]+

### Example 5

### 5-(4-(2-methyl-2-(3-isopropoxyphenyl)propoxy)phenyl)-isoxazol-3-ol

The title compound was obtained as a white solid by the method as in Example 4 using 4-(3-(methoxymethoxy)isoxazol-5-yl) phenol and
2-methyl-2-(3-(propan-2-yloxy) phenyl) propan-1-ol.
¹H-NMR(400MHz,CDCl₃)δ:7.63(2H,d,J=8.8Hz),7.27-7.22(1H,m),7.02-6.91(4H,m),6.7 6(1H,dd,J=8.4,2.0Hz),6.07(1H,s),4.55(1H,sept,J=6.2Hz),3.97(2H,s),1.46(6H,s),1.34(6H ,d,J=6.2Hz)
ESI-MS Found:m/z 368[M+H]+

### Example 6

### 5-{2-fluoro-4-[2-(3-isopropoxyphenyl)-1-methylethoxy]phenyl}isoxazol-3-ol

The title compound was obtained as a white solid by the method as in Example 4 using 4-(3-(methoxymethoxy)isoxazol-5-yl)phenol and
1-fluoro-[3-(propan-2-yloxy)phenyl]-2-propan-2-ol.
¹H-NMR(400MHz,CDCl₃)δ:7.75(1H,t,J=8.8Hz),7.23-7.17(1H,m),6.81-6.74(4H,m),6.67 (1H,dd,J=12.7,2.4Hz),6.25(1H,d,J=3.4Hz),4.61(1H,sext,J=6.3Hz),4.53(1H,sept,J=6.3Hz ),3.06(1H,dd,J=13.5,6.3Hz),2.81(1H,dd,J =13.5,6.3Hz),1.36(3H,d,J=6.3Hz),1.33(3H,d, J=6.3Hz),1.32(3H,d,J=6.3Hz).
ESI-MS Found:m/z 372[M+H]+

### Example 7

### 5-(6-(2-(3-isopropoxyphenxl)-1-methlexy)pyridin-3-yl)isoxazol-3-ol

The title compound was obtained as a white solid by the method as in Example 4 using 5-(3-(methoxymethoxy)isoxazol-5-yl)pyridine-2-ol and 1-fluoro-[3-(propan-2-yloxy)-phenyl]-propan-2-ol.
¹H-NMR(400MHz,CDCl₃)δ:8.53(1H,d,J=2.7Hz),7.86(1H,dd,J=8.8,2.7Hz),7.18(1H,t,J= 7.8Hz),6.85-6.72(4H,m),6.13(1H,s),5.49(1H,sext,J=6.5Hz),4.53(1H,sept,J=6.2Hz),3.09( 1H,dd,J=13.3,6.5Hz),2.81(1H,dd,J=13.3,6.5Hz),1.34(3H,d,J=6.2Hz),1.32(3H,d,J=6.2Hz ),1.32(3H,d,J=6.2 Hz).
ESI-MS Found:m/z 355[M+H]+

### Example 8

### 5-(5-(2-(3-isopropoxyphenyl)-1-methylethoxy)pyridin-2-yl)isoxazol-3-ol

The title compound was obtained as a white solid by the method as in Example 4 using 6-3-(-(methoxymethoxy)isoxazol-5-yl)pyridine-3-ol and 1-[3-(propan-2-yloxy)phenyl]-propan-2-ol.
¹H-NMR(400MHz,CDCl₃)δ:8.33(1H,d,J=2.6Hz),7.72(1H,d,J=8.8Hz),7.26-7.17(2H,m), 6.82-6.73(3H,m),6.43(1H,s),4.67(1H,sext,J=6.2Hz),4.53(1H,sept,J=6.1Hz),3.07(1H,dd, J=13.7,6.2Hz),2.84(1H,dd,J=13.7,6.2Hz),1.37(3H,d,J=6.1Hz),1.32(3H,d,J=6.1Hz),1.31( 3H,d,J=6.1Hz).
ESI-MS Found:m/z 355[M+H]+

### Example 9

### 5-(4-(((3-isopropoxybenzyl)(methyl)amino)methyl)phenyl)isoxazol-3-ol

To N-methyl-4-(3-(methoxymethoxy)isoxazol-5-yl)benzylamine (0.10 g), 0.075 g of 3-isopropoxybenzyl chloride and 0.17 g of potassium carbonate were added, and the mixture was stirred in dimethylformamide at 70°C overnight. The reaction solution was cooled and concentrated, and then purified by silica gel chromatography. The product was dissolved in methanol, 10% hydrochloric acid-methanol was added, and the mixture was stirred at room temperature. The concentrated reaction solution was dissolved in chloroform, the solution was washed with a sodium hydrogen carbonate solution, and drying/concentration was then carried out. The residue was purified by silica gel chromatography to yield the compound of interest as a slightly yellow amorphous substance.
¹H-NMR(400MHz,CDCl₃)δ:7.63(2H,d,J=8.8Hz),7.45-7.35(2H,m),7.26-7.18(1H,m),6.9 4-6.88(2H,m),6.80-6.75(1H,m),6.13(1H,s),4.55(1H,sept,J=6.1Hz),3.54-3.49(4H,brm),2. 19(3H,s),1.33(6H,d,J=6.1Hz)
ESI-MS Found:m/z 353[M+H]+

### Example 10

### 5-(4-(2-phenoxypropyl)phenyl)isoxazol-3-ol

### 1) Production of 1-(4-iodophenyl)propan-2-ol

A solution of iodine (3.35 g) in acetic acid (25 ml) was warmed to 100°C, concentrated sulfuric acid (3 ml) and 1-phenylpropan-2-ol (4.09 g) were then sequentially added to the solution, an aqueous solution (15 ml) containing sodium iodate (1.3 g) was further added dropwise to the mixture, and the mixture was stirred at the same temperature for 30 minutes. The mixed reaction solution was put on ice and diluted with diethyl ether and water, and the extracted organic layer was then washed with a saturated saline solution, dried over sodium sulfate, filtered and concentrated. The resultant reaction mixture was dissolved in MeOH (20 ml), 5M aqueous sodium hydroxide (5 ml) was added to the solution, and the mixture was stirred at room temperature for 1 hour and then diluted with ethyl acetate and water to extract the organic layer. The organic layer was washed with a saturated saline solution, dried over sodium sulfate, filtered and concentrated. The resultant crude product was purified by silica gel column chromatography to yield the title compound (5.1 g) as a yellow oil.

### 2) Production of 5-[4-(2-hydroxypropyl)phenyl]isoxazol-3-ol

The title compound (1.84 g) was obtained as a colorless solid by the same method as in Reference Examples 1 and 2 using 1-(4-iodophenyl)propan-2-ol as a starting material.

### 3) Production of 1-(4-(3-methoxymethoxy)isoxazol-5-yl)phenyl)propan-2-ol

To a solution of 5-(4-(2-hydroxypropyl)phenyl)isoxazol-3-ol (1.53 g) in tetrahydrofuran (18 ml), 0.54 ml of chloromethyl methyl ether and 1.00 ml of 1, 5-diazabicyclo(4,3,0)non-5-ene were added, and the reaction solution was stirred at 0°C for 5 minutes. To the reaction solution was added a saturated aqueous sodium bicarbonate solution, and the mixture was then extracted with ethyl acetate and dried over anhydrous magnesium sulfate. The solvent was removed under reduced pressure, and the resultant residue was purified by silica gel column chromatography (eluent: ethyl acetate/hexane=0/100 to 50/50) to yield the title compound (333.2 mg) as a white solid.

### 4) Production of 3-(methoxymethoxy)-5-(4-(2-phenoxypropyl)phenyl)isoxazole

To a solution of 1-(4-(3-(methoxymethoxy)isoxazol-5-yl)phenyl)propan-2-ol (50 mg) in toluene (1.0 ml), 50 µl of diisopropyl azodicarboxylate, 75 mg of triphenylphosphine and 27 µl of phenol were added, and the reaction solution was stirred at room temperature for 12 hours. The reaction solution was purified by silica gel column chromatography (eluent: ethyl acetate/hexane=0/100 to 30/70) to yield the title compound (58.8 mg) as a colorless oil.

### 5) Production of 5-(4-(2-phenoxypropyl)phenyl)isoxazol-3-ol

To a solution of 3-(methoxymethoxy)-5-(4-(2-phenoxypropyl)phenyl)isoxazole (58.8 mg) in methanol (0.5 ml), 0.5 ml of 4M hydrochloric acid-dioxane was added, and the reaction solution was stirred at room temperature for 24 hours. The solvent was removed under reduced pressure, and the resultant residue was purified by silica gel column chromatography (eluent: ethyl acetate/hexane=0/100 to 50/50) to yield the title compound (35.6 mg) as a white solid.
¹HNMR(400MHz,CDCl₃)δ:1.32(3H,d,J=5.6Hz),2.90(1H,dd,J=13.6,6.0Hz),3.10(1H,dd, J=13.6,6.0Hz),4.56-4.62(1H,m),6.16(1H,s),6.87-6.95(3H,m),7.24-7.28(2H,m),7.34(2H, d,J=8.0Hz),7.65(2H,d,J=8.4Hz)
ESI-MS Found:m/z 296[M+H]+

### Example 11

### 5-(4-(2-(3-methylphenoxy)propyl)phenyl)isoxazol-3-ol

The title compound (38.6 mg) was obtained as a white solid by the same method as in Example 10 using
1-(4-(3-(methoxymethoxy)isoxazol-5-yl)phenyl)propan-2-ol (50 mg) and m-cresol.
¹HNMR(400MHz,CDCl₃)δ:1.31(3H,d,J=6.0Hz),2.31(3H,s),2.89(1H,dd,J=13.6,5.6Hz), 3.09(1H,dd,J=14.0,6.4Hz),4.56-4.61(1H,m),6.16(1H,s),6.68-6.75(3H,m),7.14(1H,t,J=7.
2Hz),7.34(2H,d,J=8.0Hz),7.65(2H,d,J=8.4Hz)
ESI-MS Found:m/z 310[M+H]+

### Example 12

### 5-(4-(2-(3-methoxyphenoxy)propyl)phenyl)isoxazol-3-ol

The title compound (39.4 mg) was obtained as a colorless oil by the same method as in Example 10 using
1-(4-(3-(methoxymethoxy)isoxazol-5-yl)phenyl)propan-2-ol (50 mg) and 3-methoxyphenol. 1.
¹HNMR(400MHz,CDCl₃)δ:1.31(3H,d,J=6.0Hz),2.90(1H,dd,J=13.6,6.0Hz),3.09(1H,dd, J=13.6,6.0Hz),3.77(3H,s),4.56-4.61(1H,m),6.16(1H,s),6.44-6.51(3H,m),7.16(1H,t,J=8.0 Hz),7.34(2H,d,J=8.0Hz),7.65(2H,d,J=8.4Hz)
ESI-MS Found:m/z 326[M+H]+

### Example 13

### 5-(4-(2-(3-(trifluoromethxl)phenoxy)propyl)phenyl)isoxazol-3-ol

The title compound (27.3 mg) was obtained as a colorless oil by the same method as in Example 10 using
1-(4-(3-(methoxymethoxy)isoxazol-5-yl)phenyl)propan-2-ol (30 mg) and 3-(trifluoromethyl)phenol.
¹HNMR(400MHz,CDCl₃)δ:1.35(3H,d,J=6.0Hz),2.94(1H,dd,J=14.0,6.0Hz),3,10(1H,dd, J=13.6,6.4Hz),4.62-4.65(1H,m),6.18(1H,s),7.02(1H,d,J=8.4,2.0Hz),7.09(1H,brs),7.17(1 H,d,J=7.6Hz),7.33-7.38(3H,m),7.67(2H,d,J=8.0Hz)
ESI-MS Found:m/z 364[M+H]+

### Example 14

### 5-(4-(2-(4-methylphenoxy)propyl)phenyl)isoxazol-3-ol

The title compound (24.1 mg) was obtained as a colorless oil by the same method as in Example 10 using
1-(4-(3-(methoxymethoxy)isoxazol-5-yl)phenyl)propan-2-ol (30 mg) and 4-methoxyphenol.
¹HNMR(400MHz,CDCl₃)δ:1.29(3H,d,J=6.0Hz),2.87(1H,dd,J=13.6,6.0Hz),3.08(1H,dd, J=14.0,6.8Hz),3.76(3H,s),4.43-4.48(1H,m),6.17(1H,s),6.81(4H,s),7.34(2H,d,J=8.0Hz),7 .66(2H,d,J=8.4Hz)
ESI-MS Found:m/z 326[M+H]+

### Example 15

### 5-(4-(2-(3,5-dimethoxyphenoxy)propyl)phenyl)isoxazol-3-ol

The title compound (8.9 mg) was obtained as a white solid by the same method as in Example 10 using 1-(4-(3-(methoxymethoxy)isoxazol-5-yl)phenyl)propan-2-ol (16 mg) and 3,5-dimethoxyphenol.
¹HNMR(400MHz,CDCl₃)δ:1.31(3H,d,J=5.6Hz),2.90(1H,dd,J=13.6,6.0Hz),3.09(1H,dd, J=13.6,6.0Hz),3.76(6H,s),4.54-4.58(1H,m),6.07(3H,s),6.17(1H,s),7.34(2H,d,J=8.0Hz),7 .66(2H,d,J=8.0Hz)
ESI-MS Found:m/z 356[M+H]+

### Example 16

### 5-(4-(2-(4-chlorophenoxy)propyl)phenyl)isoxazol-3-ol

The title compound (13.4 mg) was obtained as a white solid by the same method as in Example 10 using
1-(4-(3-(methoxymethoxy)isoxazol-5-yl)phenyl)propan-2-ol (16 mg) and 4-chlorophenol.
¹HNMR(400MHz,CDCl₃)δ:1.31(3H,d,J=6.4Hz),2.90(1H,dd,J=14.0,6.0Hz),3.08(1H,dd, J=14.0,6.8Hz),4.52-4.56(1H,m),6.17(1H,s),6.79(2H,d,J=8.4Hz),7.21(2H,d,J=8.8Hz),7.3 3(2H,d,J=8.0Hz),7.66(2H,d,J=7.6Hz)
ESI-MS Found:m/z 330[M+H]+

### Example 17

### 5-(4-(2-(4-chloro-2-fluorophenoxy)propyl)phenyl)isoxazol-3-ol

The title compound (12.3 mg) was obtained as a colorless oil by the same method as in Example 10 using
1-(4-(3-(methoxymethoxy)isoxazol-5-yl)phenyl)propan-2-ol (16 mg) and 4-chloro-2-fluorophenol.
¹HNMR(400MH,CDCl₃)δ:1.33(3H,d,J=6.4Hz),2.93(1H,dd,J=13.6,5.2Hz),3.12(1H,dd,J =14.0,6.8Hz),4.50-4.55(1H,m),6.18(1H,s),6.82(1H,t,J=8.4Hz),6.99(1H,brd,J=8.8Hz),7. 08(1H,dd,J=10.4,2.0Hz),7.35(2H,d,J=8.0Hz),7.66(2H,d,J=7.6Hz)
ESI-MS Found:m/z 348[M+H]+

### Example 18

### 5-(4-(2-(2,4-dichlorophenoxy)propyl)phenyl)isoxazol-3-ol

The title compound (10.9 mg) was obtained as a white solid by the same method as in Example 10 using
1-(4-(3-(methoxymethoxy)isoxazol-5-yl)phenyl)propan-2-ol (16 mg) and 2, 4-dichlorophenol.
¹HNMR(400MHz,CDCl₃)δ:1.34(3H,d,J=6.0Hz),2.97(1H,dd,J=13.6,5.2Hz),3.13(1H,dd, J=13.6,6.4Hz),4.53-4.5 8(1H,m),6.18(1H,s),6.78(1H,d,J=8.8Hz),7.12(1H,dd,J=8.4,2.0Hz )7.35-7.39(3H,m),7.66(2H,d,J=7.6Hz)
ESI-MS Found:m/z 365[M+H]+

### Example 19

### 5-(4-(2-(3((trifluoromethyl)oxy)phenyl)oxy)propyl)phenyl)isoxazol-3-ol

The title compound (28 mg) was obtained as a colorless solid by the same method as in Example 10 using
1-(4-(3-(methoxymethoxy)isoxazol-5-yl)phenyl)propan-2-ol (43 mg) and 3-((trifluoromethyl))oxy)phenol.
¹H-NMR(CDCl₃)δ:7.63(2H,d,J=8.0Hz),7.30(2H,d,J=8.0Hz),7.24-7,18(1H,m),6.78-6.7 3(2H,m),6.70-6.67(1H,brm),6.14(1H,s),4.59-4.51(1H,m),3.06(1H,dd,J=13.7,6.6Hz),2.8 8(1H,dd,J=13.7,5.7Hz),1.30(3H,d,J=5.9Hz)
ESI-MS Found:m/z 380[M+H]+

### Example 20

### 5-{4-[((1R)-1-methyl-2-{3-[(1-methylethyl)]oxy]phenyl}ethyl)oxy]phenyl}isoxazol-3-o 1

### 1) Production of (2S)-1-{3-[(1-methylethyl)oxyl}propan-2-ol

To a solution of 1-bromo-3-[(1-methyl)oxy]benzene (5.38 g) in THF (50 ml), 10.34 ml of 2.66M n-butyllithium in hexane solution was added at -78°C, and the mixture was stirred for 45 minutes, followed by adding (S)-(-)-propylene oxide (2.63 ml). The mixture was stirred at -78°C for 30 minutes, followed by warming it to room temperature and further stirring it for 30 minutes. The mixture was quenched with a saturated aqueous ammonium chloride solution, diluted with water, and then extracted with ethyl acetate. The organic layer was washed with a saturated saline solution, then dried over sodium sulfate, filtered and concentrated. The resultant crude product was purified by silica gel chromatography to yield the title compound (2.2 g) as a colorless oil.

### 2) Production of

### 5-{4-[((1R)-1-methyl-2-{3-[(1-methylethyl)oxy]phenyl}ethyl)oxylphenyl}isoxazol-3-ol

The title compound (845 mg) was obtained as a colorless amorphous by the same method as in Example 4 using 4-(3-(methoxymethoxy)isoxazol-5-yl)phenol (553 mg) and (2S)-1-{3-[(1-methylethyl)oxy]}propan-2-ol.
¹H-NMR(CDCl3)δ:7.60(2H,d,J=9.0Hz),7.16(1H,dd,J=7.8,7.8Hz),6.90(2H,d,J=9.0Hz),6 .78-6.70(3H,m),6.03(1H,s),4.64-4.57(1H,m),4.52-4.46(1H,m),3.03(1H,dd,J=13.5,5.7Hz ),2.76(1H,dd,J=13.5,6.6Hz),1.30-1.27(9H,m)
ESI-MS Found:m/z 354[M+H]+

### Example 21

### 5-{4-[((1S)-1-methyl-2-{3-[(1-methylethyl)oxy]phenyl}ethyl)oxy]phenyl}isoxazol-3-ol

The title compound was obtained as a colorless amorphous by the same method as in Example 20 using (R)-(+)-propylene oxide instead of (S)-(-)-propylene oxide.

### Example 22

### 5-[4-({(1R)-1-methyl-2-[3-(cyclopropyloxy)phenyl]-1-methylethyl}oxy)phenyl]isoxazo 1-3-ol

### 1) Production of (25)-1-[3-(cyclopropyloxy)phenyl]propan-2-ol

The title compound (0.37 g) was obtained as a colorless oil by the same method as in Example 20 with 1-bromo-3-(cyclopropyloxy)benzene (0.94 g).

### 2) Production of

### 5-[4-({(1R)-1-methyl-2-[3-(cyclopropyloxy)phenyl]-1-methylethyl}oxy)phenyl]isoxazo 1-3-ol

The title compound (164 mg) was obtained as a colorless solid by the same method as in Example 4 using 4-(3-(methoxymethoxy)isoxazol-5-yl)phenol (135 mg) and (2S)-1-[3-(cyclopropyloxy)phenyl]propan-2-ol.
¹H-NMR(CDCl₃)δ:7.60(2H,d,J=9.0Hz),7.18(1H,dd,J=7.8,7.8Hz),6.93-6.87(4H,m),6.81 (1H,d,J=7.8Hz),6.03(1H,s),4.66-4.55(1H,m),3.69-3.64(1H,m),3.04(1H,dd,J=13.9,6.1Hz ),2.79(1H,dd,J=13.9,6.1Hz),1.31(3H,d,J=5.9Hz),0.74-0.70(4H,m)
ESI-MS Found:m/z 352[M+H]+

### Example 23

### 5-{4-[((1R)-1-methyl-2-{3-[(trifluoromethyl)oxy]phenyl}ethyl)oxy]phenyl}isoxazol-3-ol

### 1) Production of (2S)-1-[3-(trifluoromethyl)oxy]phenyl]propan-2-ol

The title compound (2.2 g) was obtained as a colorless oil by the same method as in Example 20 with 1-bromo-3-[(trifluoromethyl)oxy]benzene (5.0 g).

### 2) Production of 5-{4-[((1R)-1-methyl-2-{3-[(trifluoromethyl)oxy]phenyl}ethyl)oxy]phenyl}isoxazol-3-ol

The title compound (180 mg) was obtained as a colorless solid by the same method as in Example 4 using 4-(3-(methoxymethoxy)isoxazol-5-yl)phenol (111 mg) and (2S)-1-[3-[(trifluoromethyl)oxy]phenyl]propan-2-ol.
¹H-NMR(CDCl₃)δ:7.60(2H,d,J=8.6Hz),7.28(1H,dd,J=7.8,7.8Hz),7.13(1H,d,J=7.8Hz),7 .09-7.03(2H,m),6.88(2H,d,J=8.6Hz),6.03(1H,s),4.64-4.57(1H,m),3.04(1H,dd,J=13.7,6.6 Hz),2.88(1H,dd,J=13.7,5.7Hz),1.31(3H,d,J=5.9Hz)
ESI-MS Found:m/z 380[M+H]+

### Example 24

### 5-{4-[((1R)-1-methyl-2-{4-[(trifluoromethyl)oxy]phenyl}ethyl)oxy]phenyl}isoxazol-3-ol

### 1) Production of (2S)-1-{4-[(1-methylethyl)oxyl}propan-2-ol

The title compound (1.3 g) was obtained as a colorless oil by the same method as in Example 20 with 1-bromo-4-[(trifluoromethyl)oxy]benzene (2.89 g).

### 2) Production of

### 5-{4-[((1R)-1-methyl-2-{4-[(trifluoromethyl)oxy]phenyl}ethyl)oxyl]phenyl}isoxazol-3-ol

The title compound (175 mg) was obtained as a colorless amorphous by the same method as in Example 4 using 4-(3-(methoxymethoxy)isoxazol-5-yl)phenol (111 mg) and (2S)-1-[4-[(trifluoromethyl)oxy]phenyl]propan-2-ol.
¹H-NMR(CDCl₃)δ:7.60(2H,d,J=8.6Hz),7.22(2H,d,J=8.6Hz),7.11(2H,d,J=8.6Hz),6.89( 2H,d,J=8.6Hz),6.04(1H,s),4.65-4.55(1H,m),3.03(1H,dd,J=14.1,6.3Hz),2.86(1H,dd,J=14 1,5.9Hz),1.30(3H,d,J=6.3Hz)
ESI-MS Found:m/z 380[M+H]+

### Example 25

### 5-{4-[((1R)-2-{4-chloro-3-[(trifluoromethyl)oxy]phenyl}-1-methylethyl)oxylphenyl}is

### oxazol-3-ol

### 1) Production of (2S)-1-{4-chloro-3-[(trifluoromethyl)oxy]}propan-2-ol

The title compound (1.21 g) was obtained as a colorless oil by the same method as in Example 20 with 4-bromo-1-chloro-2-[(trifluoromethyl)oxy]benzene (3.18 g).

### 2) Production of

### 5-{4-{((1R)-2-{4-chloro-3-[(trifluoromethyl)oxy]phenyl}-1-methylethyl)oxy]phenyl}is oxazol-3-ol

The title compound (204 mg) was obtained as a colorless amorphous by the same method as in Example 4 using 4-(3-(methoxymethoxy)isoxazol-5-yl)phenol (111 mg) and (2S)-1-{4-chloro-3-[(trifluoromethyl)oxy]}propan-2-ol.
¹H-NMR(CDCl₃)δ:7.60(2H,d,J=9.0Hz),7.34(1H,d,J=8.2Hz),7.21-7.18(1H,brm),7.09(1 H,dd,J=8.2,2.0Hz),6.87(2H,d,J=9.0Hz),6.04(1H,s),4.63-4.55(1H,m),2.98(1H,dd,J=14.1, 7.0Hz),2.89(1H,dd,J=14.1,5.1Hz),1.31(3H,d,J=5.9Hz)
ESI-MS Found:m/z 414[M+H]+

### Example 26

### 5-{6-[((1R)-1-methyl-2-{3-[(1-methylethyl)oxy]phenyl}ethyl)oxy]pyridin-3-yl}isoxazo 1-3-ol hydrochloride

The title compound (165 mg) was obtained as colorless solid hydrochloride by the same method as in Example 4 using
5-(3-(methoxymethoxy)isoxazol-5-yl)pyridin-2-ol (111 mg) and
(2S)-1-{3-[(1-methylethyl)oxy]}propan-2-ol.
¹H-NMR(DMSO-D₆)δ:8.55(1H,d,J=2.3Hz),7.99(1H,dd,J=8.8,2.3Hz),7.10(1H,dd,J=8.0 ,8.0Hz),6.81(1H,d,J=8.8Hz),6.75-6.72(2H,m),6.69-6.65(1H,m),6.45(1H,s),5.41-5.34(1H ,m),4.53-4.46(1H,m),2.92(1H,dd,J=13.5,6.6Hz),2.79(1H,dd,J=13.5,5.7Hz),1.22(3H,d,J= 6.3Hz),1.17(6H,d,J=5.9Hz)
ESI-MS Found:m/z 355[M+H]+

### Example 27

### 5-{6-[((1S)-1-methyl-2-{3-[(1-methylethyl)oxy]phenyl}ethxl)oxy]pyridin-3-yl}isoxazol -3-ol hydrochloride

The title compound was obtained as a colorless solid by the same method as in Example 26 using (2R)-1-{3-[(1-methylethyl)oxy]}propan-2-ol instead of
(2S)-1-{3-[(1-methylethyl)oxy]}propan-2-ol.

### Example 28

### 5-{2-fluoro-4-[((1R)-1-methyl-2-{3-{(1-methylethyl)oxylphenyl}ethyl)oxy]phenyl} isox azol-3-ol

The title compound (125 mg) was obtained as a colorless oil by the same method as in Example 4 using 3-fluoro-4-(3-(methoxymethoxy)isoxazol-5-yl)phenol (120 mg) and (2S)-1-{3-[(1-methylethyl)oxy]}propan-2-ol.
¹H-NMR(CDCl₃)δ:7.73(1H,dd,J=8.6,8.6Hz),7.16(1H,dd,J=8.6,8.6Hz),6.77-6.70(4H,m) ,6.63(1H,dd,J=12.9,2.3Hz),6.23(1H,d,J=3.5Hz),5.33(2H,s),4.60-4.54(1H,m),4.52-4.45( 1H,m),3.54(3H,s),3.01(1H,dd,J=13.7,5.9Hz),2.77(1H,dd,J=13.7,6.3Hz),1.31-1.27(9H,m )
ESI-MS Found:m/z 372[M+H]+

### Example 29

### 5-{4-[(2-fluoro-2-{3-[(1-methylethyl)oxy]phenyl}ethxl)oxy]phenyl}isoxazol-3-ol

### 1) Production of 1-{3-{(1-methylethyl)oxylphenyl}-2-{[4-(3-{[(methyloxy)methylloxy}isoxazol-5-yl)ph enyl]oxy}ethanone

Reaction solution of 2-bromo-1-{3-[(1-methylethyl)oxy]phenyl}ethanone (463 mg), 4-(3-(methoxymethoxy)isoxazol-5-yl)phenol (332 mg) and potassium carbonate (331 mg) in DMF (8 ml) was stirred overnight at room temperature. The solution was quenched with a saturated aqueous sodium carbonate solution and then diluted with ethyl acetate and water. The extracted organic layer was washed sequentially with water and a saturated saline solution, dried with sodium sulfate, filtered and concentrated. The resultant crude product was purified by silica gel column chromatography to yield the title compound (500 mg) as a colorless solid.

### 2) Production of

### 1-{3-[(1-methylethyl)oxy]phenyl}-2-{[4-(3-{[(methyloxy)methyl]oxy}isoxazol-5-yl)ph enyl]oxy}ethanol

To a mixed solution of 1-{3-[(1-methylethyl)oxy]phenyl}-2-{[4-(3-{[(methyloxy)methyl]oxy}isoxazol-5-yl)ph enyl]oxy}ethanone (397 mg) in THF (4 ml) and MeOH (4 ml), sodium borohydride (57 mg) was added under ice-cooling, and the mixture was stirred at the same temperature for 30 minutes. The mixture was quenched with a saturated aqueous ammonium chloride solution and diluted with ethyl acetate and water, and the extracted organic layer was then washed with a saturated aqueous sodium chloride solution, dried over sodium sulfate, filtered and then concentrated. The resultant crude product was purified by silica gel column chromatography to yield the title compound (420 mg) as a colorless solid.

### 3) Production of

### 5-{4-[(2-fluoro-2-{3-[(1-methylethyl)oxy]phenyl}ethyl)oxy]phenyl}-3-{[(methyloxy)m ethyl]oxy}isoxazole

To a solution of 1-{3-[(1-methylethyl)oxy]phenyl}-2-{[4-(3-{[(methyloxy)methyl]oxy}isoxazol-5-yl)ph enyl]oxy}ethanol (40 mg) in dichloromethane (2 ml), DEOXO-Fluor (0.028 ml) was added under ice-cooling, and the mixture was stirred at the same temperature for 30 minutes and then purified directly by silica gel chromatography to yield the title compound (28 mg) as a colorless oil.

### 4) Production of

### 5-{4-[(2-fluoro-2-{3-[(1-methylethyl)oxy]phenyl}ethyl)oxy]phenyl}isoxazol-3-ol

The title compound (22 mg) was obtained as a colorless solid by the same method as in Example 4 with
5-{4-[(2-fluoro-2-{3-[(1-methylethyl)oxy]phenyl}ethyl)oxy]phenyl}-3-{[(methyloxy)m ethyl]oxy}isoxazole (28 mg).
¹H-NMR(CDCl₃)δ:7.63(2H,d,J=8.6Hz),7.29(1H,dd,J=8.0,8.0Hz),6.98-6.91(4H,m),6.87 (1H,dd,J=8.0,2.2Hz),6.06(1H,s),5.76(1H,ddd,J=49.0,7.8,2.7Hz),4.58-4.52(1H,m),4.36-4 26(1H,m),4.17(1H,ddd,J=28.9,10.9,2.7Hz),1.32(6H,d,J=5.9Hz)
ESI-MS Found:m/z 358[M+H]+

### Example 30

### 5-{4-[(2,2-difluoro-2-{3-[(1-methyl)oxy]phenyl}ethyl)oxy]phenyl}isoxazol-3-ol

### 1) Production of 2-bromo-1-{3-[(1-methylethyl)oxy]phenyl}propan-1-one

Reaction solution of 1-{3-[(1-methylethyl)oxy]phenyl}propan-1-one (653 mg) and copper dibromide (1.51 g) in ethyl acetate (10 ml) was stirred for 8 hours while being heated under reflux, then Celite-filtered, concentrated, and then purified by silica gel chromatography to yield the title compound (728 mg) as a colorless oil.

### 2) Production of

### 1-{3-[(1-methylethyl)oxy]phenyl}-2-{[4-(3-{[(methyloxy)methyl]oxy}isoxazol-5-yl)ph enyl]oxy}propan-1-one

The title compound (600 mg) was obtained as a colorless oil by the same method as in Example 29 with 4-(3-(methoxymethoxy)isoxazol-5-yl)phenol (332 mg) and 2-bromo-1-{3-[(1-methylethyl)oxy]phenyl}propan-1-one (488 mg).

### 3) Production of

### 5-{4-[(2,2-difluoro-1-methyl-2-{3-[(1-methylethyl)oxy]phenyl}ethyl)oxy]phenyl}-3-{[( methyloxy)methyl]oxy}isoxazole

To a solution of 1-(3-[(1-methylethyl)oxy]phenyl}-2-{[4-(3-{[(methyloxy)methyl]oxy}isoxazol-5-yl)ph enyl]oxy}propan-1-one (120 mg) in dichloromethane (4 ml), DEOXO-Fluor (0.80 ml) was added, and the mixture was stirred at room temperature for 3 days. The reaction solution was quenched with a saturated aqueous sodium bicarbonate solution and diluted with chloroform and water. The resultant extracted organic layer was dried over sodium sulfate, filtered, concentrated, and then purified by silica gel column chromatography to yield the title compound (51 mg) as a colorless oil.

### 4) Production of

### 5-{4-[2,2-difluoro-2-{3-[(1-methylthyl)oxy]phenyl}ethyl)oxy]phenyl}isoxazol-3-ol

The title compound (22 mg) was obtained as a colorless oil by the same method as in Example 4 with
5-{4-[(2,2-difluoro-1-methyl-2-{3-[(1-methylethyl)oxy]phenyl]ethyl)oxy]phenyl}-3-{[( methyloxy)methyl]oxy}isoxazole (28 mg).
¹H-NMR(CDCl₃)δ:7.59(2H,d,J=8.6Hz),7.28(1H,dd,J=8.0,8.0Hz),7.05(1H,d,J=8.0Hz),7 .03-7.01(1H,brm),6.93-6.88(3H,m),6.04(1H,s),4.78-4.67(1H,brm),4.55-4.49(1H,m),1.3 8(3H,d,J=6.3Hz),1.29(6H,dd,J=6.3,1.6Hz)
ESI-MS Found:m/z 390[M+H]

### Example 31

### 5-{4-[(2-fluoro-2-{3-[(1-methylethyl)oxy]phenyl}ethyl)oxy]phenyl}isoxazol-3-ol

The title compound was obtained as a mixture of two diastereomers by the same method as in Example 30 through the reduction, fluorination and deprotection of 1-{3-[(1-methylethyl)oxy]phenyl}-2-{[4-(3-{[(methyloxy)methyl]oxy}isoxazol-5-yl)ph enyl]oxy}propan-1-one.
¹H-NMR(CDCl₃)δ:7.64-7.59,2.0H,m),7.27-7.231.0H,m),7.01-6.79(5.0H,m),6.05-6.04( 1.0H,m),5.53(0.6H,dd,J=48.0,4.0Hz),5.40(0.4H,dd,J=46.5,6.5Hz),4.75-4.60(1.0H,m),4. 57-4.49(1.0H,m),1.36-1.28(7.8H,m),1.16(1.2H,d,J=6.6Hz)
ESI-MS Found:m/z 372[M+H]+

### Example 32

### 5-{2-[((1R)-1-methyl-2-{3-[(1-methylethl)oxy]phenyl}ethyl)oxy]pyrimidin-5-yl}isoxa zol-3-ol

### 1) Production of 5-iodo-2-[((1R)-1-methyl-2-{3-[(1-methylethyl)oxy]phenyl}ethyl)oxy]pyrimidine

To a solution of 5-iodopyrimidin-2-ol (111 mg), (2S)-1-{3-[(1-methylethyl)oxy]}propan-2-ol (194 mg) and triphenylphosphine (262 mg) in THF (5 ml), 0.19 ml of diisopropyl azodicarboxylate was added, the mixture was stirred at room temperature for 30 minutes. The reaction solution was concentrated and then purified by silica gel chromatography to yield the title compound (166 mg) as a colorless oil.

### 2) Production of ethyl-3-{2-[((1R)-1-methyl-2-{3-[(1-methylethyl)oxy]phenyl}ethyl)oxy]pyrimidin-5-yl }propyno-2-ate

The title compound (113 mg) was obtained as a colorless oil by the same method as in Reference Example 1 using 5-iodo-2-[((1R)-1-methyl-2-{3-[(1-methylethyl)oxy]phenyl}ethyl)oxy]pyrimidine (224 mg) as a starting material.

### 3) Production of

### 5-{2-[((1R)-1-methyl-2-{3-[(1-methylethyl)oxy]phenyl}ethxl)oxy]pyrimidin-5-yl}isoxa zol-3-ol

To a reaction solution of ethyl-3- {2-[((1R)-1-methyl-2-{3-[(1-methylethyl)oxy]phenyl}ethyl)oxy]pyrimidin-5-yl }propyn-2-ate (118 mg) in EtOH (4 ml) and THF (1 ml), 0.38 ml of aqueous mixed solution of hydroxylamine hydrochloride (89 mg) and 5M aqueous sodium hydroxide was added under ice-cooling, and the mixture was stirred overnight at room temperature. The mixture was quenched with 0.38 ml of 5M aqueous hydrochloric acid solution, then extracted with a mixed solvent of chloroform and methanol, dried over sodium sulfate, filtered, and then concentrated. The resultant crude product was purified by silica gel chromatography to yield the title compound (100 mg) as a colorless oil.
¹H-NMR(CDCl₃)
δ:8.79(2H,s),7.144(1H,dd,J=7.8,7.8Hz),6.83-6.77(2H,m),6.70(1H,dd,J=7.8,2.2Hz),6.19(1 H,s),5.48-5.39(1H,m),4.53-4.47(1H,m),3.13(1H,dd,J=13.5,6.3Hz),2.80(1H,dd,J=13.5,6. 8Hz), 1.36(3H,d,J=6.3Hz), 1.28(6H,d,J=5.9Hz)
ESI-MS Found:m/z 356[M+H]+

### Example 33

### 5-(4-(2-(3-isopropoxyphenyl)propoxy)phenyl)-isoxazol-3-ol

The title compound was obtained by the same method as in Example 4. ¹H-NMR(400MHz,CDCl3):δ7.62(2H,d,J=8.8Hz),7.22(1H,t,J=7.6Hz),6.91(2H,d,J=8.8H z),6.75-6.82(3H,m),6.06(1H,s),4.51-4.58(1H,m),4.05-4.14(2H,m),2.89-2.96(1H,m),1.94 -2.04(1H,m),1.62-1.73(1H,m),1.33(6H,d,J=6Hz),0.89(3H,t,J=7.0Hz).MS(M+H+):368.

### Example 34

### 5-(4-(2-(3-isopropoxyphenyl)-1-ethylethoxy)phenyl)isoxazol-3-ol

The title compound was obtained by the same method as in Example 4. ¹H-NMR(400MHz,CDCl3):δ7.60(2H,d,J=8.6Hz),7.17(1H,t,J=7.8Hz),6.91(2H,d,J=8.6H z),6.72-6.79(3H,m),6.06(1H,s),4.51-4.53(2H,m),2.96-3.0(1H,m),2.82-2.87(1H,m, H),1. 64-1.76(2H,m),1.30(6H,t,J=5.5Hz),0.98(3H,t,J=7.0Hz).
MS(M+H+):368.

### Example 35

### 5-(4-(2-(3-ethylphenyl)-1-methylethoxy)phenyl)isoxazol-3-ol

The title compound was obtained by the same method as in Example 4.
¹H-NMR(400MHz,CD30D):δ7.70(1H,d,J=8.4Hz,),7.13(1H,t,J=7.6Hz),7.01(1H,d,J=2. 4Hz),6.93(1H,dd,J=2.4Hz,J=8.4Hz),6.76-6.73(2H,m),6.69-6.73(1H,m),6.38(1H,s),4.75-4.78(1H,m),3.96(2H,q,J=7.2Hz),2.93-2.99(1H,m),2.80-2.86(1H,m),1.32(3H,t,J=7.2Hz), 1.29(3H,d,J=6.4Hz).
ESI-MS Found:m/z 374(M+H+).

### Example 36

### 5-(4-(2,2-difluoro-2-(3-isopropoxyphenyl)ethoxy)phenyl)isoxazol-3-ol

The title compound was obtained by the same method as in Example 30 using 1- {3-[(1-methylethyl)oxy]phenyl}ethan-1-one instead of
1- {3-[(1-methylethyl)oxy]phenyl}propan-1-one.
¹H-NMR(400MHz,CD30D):δ7.66(2H,d,J=8.0Hz),7.36(1H,t,J=8.0Hz),7.13(2H,d,J=8.0 Hz),7.00-7.03(3H,m),6.17(1H,s),4.60-4.62(1H,m),4.51(2H,t,J=12.4Hz),1.30(6H,d,J=6.0 Hz).
ESI-MS Found:m/z 376(M+H+).

### Example 37

### 5-{2-fluoro-4-[2-(3-ethoxyphenyl)-1-methylethoxy]phenyl}isoxazol-3-ol

The title compound was obtained by the same method as in Example 4 using 2-fluoro-4-(3-(methoxymethoxy)isoxazol-5-yl)phenol and
1-(3-ethoxyphenyl)propan-2-ol.
¹H-NMR(400MHz,CDCl3):δ7.74(1H,t,J=8.6Hz),7.20(1H,t,J=8.6Hz),6.66-6.81(5H,m), 6.24(1H,d,J=3.1Hz),4.57-4.65(1H,m),3.98-4.04(2H,q,J=7.0Hz),3.03-3.08(1H,m),2.79-2. 84(1H,m),1.40(3H,t,J=7.0Hz),1.32(3H,d,J=6.3Hz).
ESI-MS Found:m/z 358[M+H]+

### Example 38

### 5-{2-fluoro-4-[2-(3-isopropoxyphenyl)-2-methylpropoxy]phenyl}isoxazol-3-ol

The title compound was obtained by the same method as in Example 4 using 2-fluoro-4-(3-(methoxymethoxy)isoxazol-5-yl)phenol and
2-methyl-2-[3-(propan-2-yloxy)phenyl]propan-1-ol.
¹H-NMR(400MHz,CDCl3):δ7.74(1H,t,J=8.6Hz),7.24(1H,t,J=8.6Hz),6.95-6.98(2H,m), 6.75-6.77(2H,m),6.66(1H,d,J=12.5Hz),6.24(1H,d,J=3.1Hz),4.50-4.61(1H,m),3.95(2H,s) ,1.44(6H,s), 1.33(6H,d,J=6.3Hz)
ESI-MS Found:m/z 386[M+H]+

### Example 39

### 5-{2-fluoro-4-[2-(3-isopropoxyphenyl)propoxy]phenyl}isoxazol-3-ol

The title compound was obtained by the same method as in Example 4 using 2-fluoro-4-(3-(methoxymethoxy)isoxazol-5-yl)phenol and
2-[3-(propan-2-yloxy)phenyl]propan-1-ol.
¹H-NMR
(400MHz,CDCl3):δ7.75(1H,t,J=8.6Hz),7.23(1H,t,J=7.8Hz),6.66-6.85(5H,m),6.25(1H,d, J=3.9Hz),4.51-4.60(1H,m),4.08-4.13(1H,m),3.95-4.0(1H,m),3.16-3.25(1H,m),1.39(3H, d,J=6.3Hz),1.33 (6H,d,J=6.3Hz).
ESI-MS Found:m/z 372[M+H]+

### Example 40

### 5-(6-(2-(3-isopropoxyphenyl)propoxy)pyridin-3-yl)isoxazol-3-ol

The title compound was obtained by the same method as in Example 4 using 5-(3-(methoxymethoxy)isoxazol-5-yl)pyridin-2-ol and
2-[3-(propan-2-yloxy)phenyl]propan-1-ol.
¹H-NMR(400MHz,CD30D):δ8.51(s,1H),7.97(d,J=8.4Hz,1H),7.17(t,J=8.4Hz,1H),6.80-6.84(m,3H),6.71-6.76(m,1H),6.26(s,1H),4.52-4.58(m,1H),4.39-4.47(m,1H),4.34-4.37(m ,1H),3.24-3.34(m,1H),1.37(d,J=6.8Hz,3H),1.28(d,J=6.0Hz,6H).
ESI-MS Found:m/z 355[M+H]+

### Example 41

### 5-(4-{[[1-(3-isopropoxyphenyl)ethyl(methyl)amino]methyl}phenyl)isoxazol-3-ol

The title compound was obtained by the same method as in Example 9 using N-methyl-4-(3-(methoxymethoxy)isoxazol-5-yl)benzylamine (0.10 g) and
1-(1-chloroethyl)-3-(propan-2-yloxy)benzene.
¹H-NMR(400MHz,MeOH):δ7.85(2H,d,J=8.6Hz),7.50-7.60(2H,br),7.42(1H,t,J=7.8Hz), 7.00-7.10(3H,m),6.40(1H,s),4.40-4.70(1H,m),4.0-4.10(1H,br),2.60-2.80(3H,br),1.80-1. 90(3H,br),1.30(6H,d,J=6.3Hz).
ESI-MS Found:m/z 367[M+H]+

### Example 42

### 2-[4-(3-hydroyisoxazol-5-yl)phenoxy]-1-(3-isopropoxyphenyl)ethanone

The title compound was obtained by deprotection of 1-{3-[(1-methylethyl)oxy]phenyl}-2-{[4-(3-{[(methyloxy)methyl]oxy}isoxazol-5-yl)ph enyl]oxy}ethanone, obtained in Example 29-1), in accordance with the method of Example 4.
¹H-NMR(400MHz,CD30D):δ7.68(2H,d,J=8.0Hz),7.61(1H,d,J=8.0Hz),7.51(1H,s),7.43 (1H,t,J=8.0Hz),7.29(1H,d,J=8.0Hz),7.06(2H,d,J=8.0Hz),6.75-6.85(1H,br),5.50(2H,s),4. 65-4.69(1H,m),1.29(6H,d,J=6.0Hz).
ESI-MS Found:m/z 354[M+H]+

### Example 43

### 2-[2-(3-ethoxyphenyl)-1-methylethoxy]-5-(3-hydroxyisoxazol-5-yl)pyridinium trifluoroacetate

The reaction mixture was obtained by the same method as in Example 4 using 5-(3-(methoxymethoxy)isoxazol-5-yl)pyridine-2-ol and 1-(3-ethoxyphenyl)propan-2-ol and was purified by reversed phase chromatography (0.1 % TFA-containing acetonitrile-water system) to yield the title compound. ¹H-NMR (400 MHz, CD3OD):
δ8.51(1H,s),7.97(1H,d,J=8.4Hz,),7.13(1H,t,J=8.4Hz),6.79-6.82(3H,m),6.69-6.72(1H,m) ,6.26(1H,s),5.42-5.47(1H,m),3.96(2H,q,J=6.8Hz),3.01-3.06(1H,m),2.80-2.85(1H,m),1.2 9-1.36(6H,m).
ESI-MS Found:m/z 341 [M+H]+

### Example 44

### 5-(3-hydroxyisoxazol-5-yl)-2-[2-(3-isopropoxyphenyl)-2-methylpropoxy]pyridinium trifluoroacetate

The title compound was obtained by the same method as in Example 4 using 5-(3-(methoxymethoxy)isoxazol-5-yl)pyridine-2-ol and 2-methyl-2-[3-(propane-2-yloxy)phenyl]propan-1-ol.
¹H-NMR(400MHz,CD30D):δ8.51(1H,s),7.97(1H,d,J=8.4Hz),7.18(1H,t,J=8.4Hz),6.94-6.99(2H,m),6.82(1H,d,J=8.4Hz),6.81(1H,d,J=8.4Hz),6.26(1H,s),4.52-4.58(1H,m),4.38( 2H,s),1.42(6H,s),1.27(6H,d,J=6.0Hz).
ESI-MS Found:m/z 369[M+H]+

### Example 45

### 3-{2-[4-(3-hydroxyisoxazol-5-yl)phenoxy]propyl}-N,N-dimethylaniline hydrochloride

The title compound was obtained by the same method as in Example 4 using 4-(3-(methoxymethoxy)isoxazol-5-yl)phenol and
1-[3-(dimethylamino)phenylpropan-2-ol.
¹H-NMR(400MHz,CD30D):δ7.59-7.63(3H,m),7.48-7.51(3H,m),6.95(2H,d,J=8.0Hz),6. 13(1H,s),4.77-4.97(1H,m),3.23-3.33(6H,m),3.07(2H,d,J=8.0Hz),1.35(3H,d,J=8.0Hz). ESI-MS Found:m/z 339[M+H]+

### Example 46

### 2-[4-(3-hydroyisoxazol-5-yl)phenoxy]-1-(3-isopropoxyphenyl)propan-1-one

The title compound was obtained by deprotection of a synthesized intermediate product of Example 30,
1-{3-[(1-methylethyl)oxy]phenyl}-2-{[4-(3-{[(methyloxy)methyl]oxy}isoxazol-5-yl)ph enyl]oxy}propan-1- one, by the method of Example 4.
¹H-NMR
(400MHz,CDCl3):δ7.48-7.55(3H,m),7.47(1H,s),7.30(1H,t,J=8.0Hz),7.03-7.06(1H,m),6. 82-6.85(2H,m),5.97(1H,s),5.49-5.51(1H,q,J=8.0Hz),4.49-4.55(1H,m),1.66(3H,d,J=8.0H z),1.27(6H,d,J=8.0Hz).
ESI-MS Found:m/z 368[M+H]+

### Example 47

### 5-(4-{[1-(3-isopropoxyphenyl)ethoxy]methyl}phenyl)isoxazol-3-ol

To a solution of 3-isopropoxyacetophenone (100 mg) and FeCl3 (3.5 mg) in nitromethane (2 ml), 124 mg of 4-(3-(methoxymethoxy)-5-isoxazolyl)phenol and 61 mg of triethylsilane were added under ice-cooling, and the mixture was stirred. A phosphate buffer was added to the reaction solution, the mixture was extracted with chloroform, and the organic layer was washed with a saturated saline solution, dried over anhydrous sodium sulfate, and concentrated. The residue was purified with preparative TLC to give
5-(4-{[1-(3-isopropoxyphenyl)ethoxy]methyl}phenyl)-3-(methoxymethoxy)isoxazole, which was deprotected in accordance with the method of Example 4 to yield the compound of interest.
¹H-NMR(400MHz,CD3OD):δ7.72(2H,d,J=8.0Hz),7.40(2H,d,J=8.0Hz),7.23(1H,t,J=8.0 Hz),6.88(2H,d,J=8.0Hz),6.79(1H,t,J=8.0Hz),6.28(1H,s),4.58-4.62(H,m),4.46-4.57(1H, m),4.39(2H,m), 1.43(3H,d,J=6.4Hz), 1.29(6H,d,J=6.0Hz).
ESI-MS Found:m/z 354[M+H]+

### Example 48

### N-{2-[4-(3-hydroxyisoxazol-5-yl)phenyl]ethyl}-3-isopropoxy-N-methylaniline trifluoroacetate

A mixture of 2-[4-(3-hydroxyisoxazol-5-yl)phenyl]ethyl methanesulfonate (100 mg), N-methyl-3-isopropoxyaniline (56 mg), potassium iodide (5 mg) and acetonitrile (4 ml) was heated to 110°C in a micro-wave reactor for 20 minutes. The reaction mixture was concentrated and purified by reverse phase HPLC (0.1 % TFA-containing acetonitrile-water system) to yield the title compound.
¹H-NMR(400MHz,CD3OD):δ7.68(2H,d,J=7.8Hz),7.31(2H,d,J=7.8Hz),7.21(1H,t,J=7.8 Hz),6.46-6.60(3H,m),6.26(1H,s),4.51-4.59(1H,m),3.68(2H,t,J=7.0Hz),2.99(3H,s),2.88(2 H,t,J=7.0Hz),1.28(6H,d,J=6.3Hz).
ESI-MS Found:m/z 353[M+H]+

### Example 49

### 1-[4-(3-hydroxyisoxazol-5-yl)phenyl]-N-(3-isopropoxybenzyl)-N-methylethanamine trifluoroacetate

Instead of N-methyl-4-(3-(methoxymethoxy)isoxazol-5-yl)benzylamine, N,α-dimethyl-4-(3-(methoxymethoxy)isoxazol-5-yl)benzylamine was used by the same method as in Example 9, and further purified by reverse phase HPLC (0.1 % TFA-containing acetonitrile-water system) to yield the title compound.
¹H-NMR(400MHz,CD30D):δ7.95(2H,d,J=8.6Hz),7.60-7.70(2H,br),7.40(1H,t,J=8.6Hz ),7.05(1H,d,J=7.8Hz),6.95(2H,t,J=7.8Hz),6.42(1H,s),4.50-4.70(2H,m),4.45(2H,s),2.60-2.80(3H,m),1.80-1.90(3H,br),1.30(6H,d,J=6.3Hz).
ESI-MS Found:m/z 367[M+H]+

### Example 50

### 5-[4-({[1-(3-isopropoxyphenyl)ethyl]thio}methyl)phenyl]isoxazol-3-ol

Using 4-(3-(methoxymethoxy)isoxazol-5-yl)benzylthiol instead of N-methyl-4-(3-(methoxymethoxy)isoxazol-5-yl)benzylamine and using 3-isopropoxy-α-methylbenzyl chloride instead of 3-isopropoxybenzyl chloride, the title compound was obtained by the same method as in Example 9.
¹H-NMR:(400MHz,CD30D):δ7.65(2H,d,J=8.6Hz),7.30(2H,d,J=8.6Hz),7.20(1H,t,J=8. 0Hz),6.70-6.90(3H,m),6.25(1H,s),4.50-4.60(1H,m),3.80(1H,q,J=7.0Hz),3.50-3.60(2H, m),1.50(3H,d,J=7.0Hz),1.30(6H,d,J=6.3Hz).
ESI-MS Found:m/z 370[M+H]+

### Example 51

### N-[4-(3-hydroxyisoxazol-5-yl)benzyl]-3-isopropoxy-N-methylbenzenesulfonamide

To a solution of N-methyl-4-(3-(methoxymethoxy)isoxazol-5-yl)benzylamine (100 mg) in dichloromethane (6 ml), 100 mg of 3-isopropoxy-benzenesulfonamide and 81 mg of triethylamine were added, and the mixture was stirred overnight at room temperature and concentrated. The residue was purified by preparative TLC to give 3-isopropoxy-N- {4[3-(methoxymethoxy)isoxazol-5-yl]benzyl}-N-methylbenzenesulfon amide. To the product was added 1N hydrochloric acid/methanol, the mixture was stirred at room temperature, and the deprotection of the MOM group was confirmed to concentrate the mixture. The title compound was obtained by purifying the residue by silica gel chromatography.
¹H-NMR(400MHz,CD30D):δ7.71(2H,d,J=8.0Hz),7.49(1H,t,J=8.0Hz),7.34-7.42(3H,m ),7.25(1H,d,J=4.4Hz),7.16-7.19(1H,m),6.30(1H,s),4.62-4.68(1H,m),4.18(2H,s),2.60(3H ,s),1.30(6H,d,J=6.0Hz).
ESI-MS Found:m/z 381[M+H]+

### Example 52

### 5-{4-[2-(3-isopropoxyphenoxy)propyl]phenyl}isoxazol-3-ol

The title compound was obtained by the same method as in Example 10 using 3-isopropoxyphenol instead of phenol.
¹H-NMR(400MHz,CD30D):δ7.63-7.66(2H,m),7.35(2H,d,J=8.4Hz),7.08(lH,t,J=8.0Hz ),6.41-6.45(2H,m),6.33(1H,t,J=2.4Hz),6.24(1H,s),4.56-4.64(1H,m),4.44-4.50(1H,m),2.9 9-3.03(1H,m),2.87-2.92(1H,m),1.23-1.27(9H,m).
ESI-MS Found:m/z 354[M+H]+

### Example 53

### 5-{4-[2-(3-isobutylphenyl)-1-methylethoxy]phenyl}isoxazol-3-ol

The title compound was obtained by the same method as in Example 4.
¹H-NMR(400MHz,CDCl3):δ7.77(1H,bs),7.66(2H,d,J=8.8Hz),7.23(1H,t,J=7.6Hz),7.01-7.10(3H,m),6.96(2H,d,J=8.8Hz),6.11(1H,s),4.62-4.73(1H,m),3.07-3.15(1H,m),2.81-2.9 0(1H,m),2.47(2H,d,J=6.8Hz),1.79-1.95(1H,m),1.36(3H,d,J=6.0Hz),0.91(6H,d,J=7.2Hz)
ESI-MS Found:m/z 352[M+H]+

### Example 54

### 5-{3-fluoro-4-[2-(3-isopropoxyphenyl)-1-methylethoxy]phenyl}isoxazol-3-ol

The title compound was obtained by the same method as in Example 4. ¹H-NMR(400MHz,CD30D):δ7.34(1H,t,J=8.4Hz),7.17-7.21(1H,m),6.65-6.79(5H,m),6. 25(1H,s),4.49-4.63(2H,m),3.02-3.07(1H,m),2.78-2.83(1H,m),1.25-1.42(9H,m). ESI-MS Found:m/z 372[M+H]+

### Example 55

### 5-(4-{2-[(3-isopropoxyphenyl)thio]propyl}phenyl)isoxazol-3-ol

The title compound was obtained by the same method as in Example 9 using 3-isopropoxythiophenol and 1-(4-(3-(methoxymethoxy)isoxazol-5-yl)phenyl)prop-2-yl methanesulfonate instead of N-methyl-4-(3-(methoxymethoxy)isoxazol-5-yl)benzylamine and 3-isopropoxybenzyl chloride.
¹H-NMR(400MHz,CD30D):δ7.65(2H,d,J=7.6Hz),7.29(2H,d,J=7.6Hz),7.16(1H,t,J=7.6 Hz),6.93(1H,d,J=7.6Hz),6.84-6.86(1H,m),6.73-6.76(1H,m),6.24(1H,s),4.54-4.56(1H,m) ,3.46-3.54(1H,m),2.93-2.99(1H,m),2.73-2.78(1H,m),1.27(6H,q,J=6.4Hz),1.24(3H,d,J=7 .2Hz).
ESI-MS Found:m/z 370[M+H]+

### Example 56

### 5-{4-[2-(3-ethoxyphenyl)-1-methylethoxy]-3-fluorophenyl}isoxazol-3-ol

The title compound was obtained by the same method as in Example 4.
¹H-NMR(400MHz,CD30D):δ7.41-7.47(2H,m),7.06-7.15(2H,m),6.79-6.82(2H,m),6.71 (1H,d,J=7.2Hz),6.19(1H,s),4.68-4.59(1H,m),3.97(2H,q,J=6.8Hz),2.97-3.02(1H,m),2.83-2.87(1H,m),1.29-1.35(6H,m).
ESI-MS Found:m/z 358[M+H]+

### Example 57

### 5-{4-[2,2-difluoro-2-(3-isopropoxyphenyl)ethoxy]-2-fluorophenyl}isoxazol-3-ol

To a solution of 5- {4-[2-oxo-2-(3-isopropoxyphenyl)ethoxy]-2-fluorophenyl}-3-methoxymethoxyisoxaz ole (200 mg) in dichloromethane (2 mL), BAST (320 mg) was added at 0°C, and the mixture was heated to reflux overnight. The reaction mixture was left standing to cool, a saturated saline solution was added, and the mixture was extracted with dichloromethane. The organic layer was concentrated, the residue was purified with preparative TLC (petroleum ether:ethyl acetate=5:1) to give
5- {4-[2,2-difluoro-2-(3-isopropoxyphenyl)ethoxy]-2-fluorophenyl}-3-methoxymethoxy isoxazole, which was deprotected by the same method as in Example 4 to yield the title compound.
¹H-NMR(400MHz,CD30D):δ7.72(1H,t,J=8.0Hz),7.34(1H,t,J=8.0Hz),7.10(1H,d,J=8.0 Hz),7.06(1H,s),6.99(1H,d,J=8.0Hz),6.83-6.87(2H,m),6.16(1H,s),4.56-4.63(1H,m),4.51( 2H,t,J=12.4Hz),1.27(6H,d,J=6.4Hz).
ESI-MS Found:m/z 394[M+H]+

### Example 58

### 5-(4-{2-[(3-isopropoxyphenyl)(methyl)amino]propyl}phenyl)isoxazol-3-ol

### 1) Production of N-[2-(4-iodophenyl)-1-methylethyl]-3-isopropoxy-N-methylaniline

To a solution of N-[2-(4-iodophenyl)-1-methylethyl]-3-isopropoxyaniline (770 mg) in DMF (10 ml), sodium hydride (230 mg) was added. The mixture was stirred at room temperature for 30 minutes, followed by adding methyl iodide (830 mg) thereto and stirring the mixture at 50°C overnight. The reaction mixture was cooled, water was added, and the mixture was extracted with ethyl acetate. The organic layer was concentrated to give N-[2-(4-iodophenyl)-1-methylethyl]-3-isopropoxy-N-methylaniline (750 mg).

### 2) Production of ethyl

### 3-(4-{2-[(3-isopropoxyphenyl)(methyl)amino]propyl}phenyl)prop-2-enoate

The mixture of N-[2-(4-iodophenyl)-1-methylethyl]-3-isopropoxy-N-methylaniline (750 mg), HCCCO₂Et (477 mg), Cu₂O (278 mg) and DMF (20 ml) was stirred at 100°C overnight. The reaction mixture was filtered through Celite, and the filtrate was concentrated and purified by preparative TLC (petroleum ether/ethyl acetate=10:1) to give the compound of interest (160 mg).

### 3) Production of

### 5-(4-{2-[(3-isopropoxyphenyl)(methyl)amino]propyl}phenyl)isoxazol-3-ol

To 10% NaOH aqueous solution (2 mL), hydroxylamine hydrochloride (0.11 g) was added at 0°C, MeOH (10 mL) and
5-(4- {2-[(3-isopropoxyphenyl)(methyl)amino]propyl}phenyl)isoxazol-3-ol (150 mg) were further added, and the mixture was stirred overnight at room temperature. To the reaction solution was added 10% hydrochloric acid to adjust the pH to 2, and a saturated saline solution was added to the mixture, which was extracted with dichloromethane. The organic layer was concentrated, and the residue was purified by preparative TLC (petroleum ether/ethyl acetate= 5:1) to yield the title compound.
¹H-NMR(400MHz,CDCl₃):δ7.58(2H,d,J=8.4Hz),7.23(2H,d,J=8.4Hz),7.05(1H,t),6.30 -6.28(1H,m),6.21-6.19(2H,m),6.09(1H,s),4.46-4.41(1H,m),4.11-4.06(1H,m),2.92-2.87(1 H,m),2.70-2.66(1H,m),2.70(3H,s),1.29(6H,d,J=6Hz),1.11(3H,d,J=6.8Hz).
ESI-MS Found:m/z 367[M+H]+

### Example 59

### 4-(3-hydroxyisoxazol-5-yl)-N-[2-(3-isopropoxyphenyl)-1-methylethyl]aniline trifluoroacetate

N-[2-(3-isopropoxyphenyl)-1-methylethyl]-4-[3-(methoxymethoxy)isoxazol-5-yl]aniline was deprotected by the same method as in Example 4 and purified by reverse phase HPLC (0.1% TFA-containing acetonitrile-water system) to yield the title compound.
¹H-NMR(400MHz,CD30D):δ7.59(2H,d,J=8.8Hz),7.14(1H,t,J=7.6Hz),6.87(2H,d,J=8. 8Hz),6.68-6.76(3H,m),6.07(1H,s),4.46-4.55(1H,m),3.76-3.84(1H,m),2.88-2.93(1H,m),2 .64-2.69(1H,m),1.23(6H,d,J=6.4Hz),1.15(3H,d,J=6.4Hz).
ESI-MS Found:m/z 353[M+H]+

### Example 60

### 4-(3-hydroxyisoxazol-5-yl)-N-[2-(3-isopropoxyphenyl)ethyl]-N-methylaniline trifluoroacetate

N-[2-(3-isopropoxyphenyl)ethyl]-4-[3-(methoxymethoxy)isoxazol-5-yl]-N-met hylaniline was deprotected by the same method as in Example 4 and purified by reverse phase HPLC (0.1% TFA-containing acetonitrile-water system) to yield the title compound.
¹H-NMR(400MHz,CD30D):δ7.53(2H,d,J=8.8Hz),7.13(1H,t,J=7.6Hz),6.68-6.76(5H, m),5.98(1H,s),4.45-4.54(1H,m),3.62(2H,t,J=7.2Hz),2.88(3H,s),2.81(2H,t,J=7.2Hz),1.23 (6H,d,J=6.4Hz).
ESI-MS Found:m/z 353[M+H]+

### Example 61

### 5-{4-[2-(3-isopropoxyphenyl)-1-methylethoxy]-3-methoxyphenyl}isoxazol-3-ol

The title compound was obtained by the same method as in Example 4. ¹H-NMR(400MHz,CDCl3):δ7.09-7.28(3H,m),6.66-6.84(4H,m),6.02(1H,s),4.44-4.55( 2H,m),3.84(3H,s),2.85-3.10(2H,m),1.20-1.34(9H,m).
ESI-MS Found:m/z 384[M+H]+

### Example 62

### 5-(4-(1-methyl-2-((1-methylethyl)oxy)pyridin-4-yl)ethyl)oxy)phenyl)isoxazol-3-ol hydrochloride

2-((1-methylethyl)oxy)-4-(2-((4-(3-(((methyloxy)methyl)oxy)isoxazol-5-yl)phenyl)oxy) propyl)pyridine (16 mg) obtained in Reference Example 9 was dissolved in 10% hydrochloric acid-methanol (1 mL), and the solution was stirred at room temperature for 2 hours. The reaction solution was removed under reduced pressure to yield the hydrochloride of the title compound as a pale yellow oil.
¹H-NMR(400MHz,DMSO-d6)δ:8.03(1H,d,J=5.4Hz),7.68(2H,d,J=9.0Hz),7.04(2H,d,J= 9.0Hz),6.89(1H,d,J=5.4Hz),6.70(1H,s),6.37(1H,s),5.23-5.13(1H,m),4.91-4.83(1H,m),2. 95(1H,dd,J=13.8,6.0Hz),2.86(1H,dd,J=13.8,6.0Hz),1.25(6H,d,J=6.0Hz),1.24(3H,d,J=6. 0Hz).
ESI-MS Found:m/z 355[M+H]+

### Example 63

### 5-(4-(((1S)-1-methyl-2-(2-((1-methylethyl)oxy)pyridin-4-yl)ethyl)oxy)phenyl)isoxazol-3-ol sodium salt

2-((1-methylethyl)oxy)-4-(2-((2S)-2-((4-(3-(((methyloxy)methyl)oxy)isoxazol-5-yl)phe nyl)oxy)propyl)pyridine (84 mg) obtained in Reference Example 10 was dissolved in 10% hydrochloric acid-methanol (5 mL), and the solution was stirred at room temperature for 2 hours. The reaction liquid was removed under reduced pressure and purified by silica gel column chromatography (eluent: methanol/chloroform=1/99 to 10/90) to give a colorless oil, which was dissolved in water (2 mL), and 1N NaOH water (0.40 mL) was added. This solution was purified using an ODS column (SepPak manufactured by Waters, C18, 1 g, eluent: methanol/water=0/100 to 50/50) to give the sodium salt of the title compound as a colorless solid.
¹H-NMR(400MHz,DMSO-D6)δ:8.01(1H,dJ=5.4Hz),7.44(2H,d,J=8.8Hz),6.93(2H,d,J= 8.8Hz),6.86(1H,d,J=5.4Hz),6.64(1H,s),5.54(1H,s),5.22-5.16(1H,m),4.82-4.74(1H,m),2. 92(1H,dd,J=14.0,6.0Hz),2.82(1H,dd,J=14.0,6.0Hz),1.25(6H,d,J=6.0Hz),1.21 (3H,d,J=6. 0Hz).
ESI-MS Found:m/z 355[M+H]+

### Example 64

### 5-(4-(((1R)-1-methyl-2-(2((1-methylethyl)oxy)pyridin-4-yl)ethyl)oxy)phenyl)isoxazol-3-ol sodium salt

The sodium salt of the title compound was obtained as a colorless solid by the same process as in Example 2 using 2-((1-methylethyl)oxy)-4-((2R)-2-((4-(3-(((methyloxy)methyl)oxy)isoxazol-5-yl)phenyl )oxy)propyl)pyridine (80 mg), obtained in Reference Example 10, as a starting material. ¹H-NMR(400MHz,DMSO-D6)δ:8.01(1H,dJ=5.4Hz),7.44(2H,d,J=8.8Hz),6.93(2H,d,J= 8.8Hz),6.86(1H,d,J=5.4Hz),6.64(1H,s),5.54(1H,s),5.22-5.16(1H,m),4.82-4.74(1H,m),2. 92(1H,dd,J=14.0,6.0Hz),2.82(1H,dd,J=14.0,6.0Hz),1.25(6H,d,J=6.0Hz),1.21(3H,d,J=6. 0Hz).
ESI-MS Found:m/z 355[M+H]+

### Example 65

### N-[4-(3-hydroxyisoxazol-5-yl)benzyl]-3-isopropoxy-N-methylaniline

To a solution of N-methyl-4-[3-(methoxymethoxy)isoxazol-5-yl]benzylamine (100 mg) in dichloromethane (6 mL), 3-isopropoxybenzoyl chloride (95 mg) and triethylamine (81 mg) were added, and the mixture was stirred overnight. The reaction solution was concentrated and purified by preparative TLC to give 3-isopropoxy-N-{4-[3-(methoxymethoxy)isoxazol-5-yl]benzyl}-N-methylbenzamide, which was deprotected in accordance with the method of Example 4 to yield the title compound.
¹H-NMR(400MHz,DMSO-d6):δ7.75(2H,d,J=7.8Hz),7.20-7.50(3H,m),6.80-7.0(3H,m), 6.50(1H,s),4.40-4.70(3H,m),2.80-2.90(3H,m),1.10-1.30(6H,m).
ESI-MS Found:m/z 367[M+H]+

### Reference Example 1

### ethyl 3-(4-hydroxyphenyl)-2-propinoate

To a solution of 4-iodophenol (22.3 g) in dimethylformamide (200 ml), 19.5 ml of ethyl propiolate and 14.8 g of copper (I) oxide were added, and the reaction solution was stirred at 110°C for 15 hours. The reaction solution was cooled and then filtered through Celite, and the filtrate was concentrated under reduced pressure. The resultant residue was purified by silica gel column chromatography (eluent: hexane/ethyl acetate=4/1) and then recrystallized with hexane/ethyl acetate to yield the title compound as a colorless solid.
¹H-NMR(400MHz,CDCl₃)δ:1.36(3H,t,J=7.2Hz),4.30(2H,q,J=7.2Hz),5.86-6.25(1H,br m),6.84(2H,d,J=8.5Hz),7.46(2H,d,J=8.5Hz)
ESI-MS Found:m/z 191[M+H]+

### Reference Example 2

### 4-(3-(methoxymethoxy)-5-isoxazolyl)phenol

### 1) Production of ethyl 3-(4-(benzyloxy)phenyl)-2-propinoate

To a solution of ethyl 3-(4-hydroxyphenyl)-2-propinoate (1.02 g), obtained in Reference Example 1, in acetone (25 ml), 3.69 g of potassium carbonate and 0.64 ml of benzylbromide were added, and the reaction solution was stirred at 65°C for 4 hours. The reaction solution was cooled, thereafter water was added, and the mixture was extracted with chloroform and dried over anhydrous sodium sulfate. The solvent was removed under reduced pressure, and the resultant residue was purified by silica gel column chromatography (eluent: ethyl acetate/hexane=10/90 to 50/50) to yield the title compound as a white solid.

### 2) Production of 5-(4-(benzyloxy)phenyl)isoxazol-3-ol

To a solution of ethyl 3-(4-(benzyloxy)phenyl)-2-propinoate (1.46 g) in methanol (30 ml), 1.45 g of hydroxyamine hydrochloride and 6.25 ml of 5M potassium hydroxide-methanol solution were added, and the reaction solution was stirred overnight at room temperature. The solvent was removed under reduced pressure, the resultant residue was suspended in water, and 2N aqueous hydrochloric acid solution was used to adjust the pH to 2 to 3. The resultant solid was obtained through filtration to give the title compound as a light brown solid.

### 3) Production of 5-(4-(benzyloxy)phenyl)-3-(methoxymethoxy)isoxazole

To a solution of 5-(4-(benzyloxy)phenyl)isoxazol-3-ol (1.38 g) in tetrahydrofuran (30 ml), 2.15 ml of triethylamine and 0.51 ml of methoxymethyl chloride were added, and the reaction solution was stirred overnight at room temperature. To the reaction solution was added a saturated aqueous ammonium chloride solution, and the mixture was extracted with ethyl acetate. The combined organic layers were washed with a saturated saline solution and dried over anhydrous sodium sulfate. The solvent was removed under reduced pressure, and the resultant residue was purified by silica gel column chromatography (eluent: ethyl acetate/hexane=10/90 to 0/100) to yield the title compound as a light brown solid.

### 4) Production of 4-(3-(methoxymethoxy)isoxazol-5-yl)phenol

To a mixed solution of 5-(4-(benzyloxy)phenyl)-3-(methoxymethoxy)isoxazole (1.44 g) in methanol (15 ml) and tetrahydrofuran (15 ml), 200 mg of 10% palladium-carbon catalyst was added, and the reaction solution was stirred overnight under hydrogen atmosphere. The reaction solution was filtered through Celite, the solvent was removed under reduced pressure, and the resultant residue was purified by silica gel column chromatography (eluent: ethyl acetate/hexane=10/90 to 50/50) to yield the title compound as a pale yellow solid.
¹H-NMR(400MHz,CDCl₃)δ:3.58(3H,s),5.35(2H,s),6.11(1H,s),6.40(1H,brs),6.92-6.96( 2H,m),7.61-7.64(2H,m)
ESI-MS Found:m/z 222[M+H]+

### Reference Example 3

### 1-(4-iodophenyl)propan-2-ol

A solution of iodine (3.35 g) in acetic acid (25 ml) was warmed to 100°C, concentrated sulfuric acid (3 ml) and 1-phenylpropan-2-ol (4.09 g) were then sequentially added, an aqueous solution (15 ml) containing sodium iodate (1.3 g) was further added dropwise, and the mixture was stirred at the same temperature for 30 minutes. The mixed reaction solution was put on ice, diluted with diethyl ether and water, and thereafter the extracted organic layer was washed with a saturated saline solution, dried over sodium sulfate, filtered and concentrated. The resultant reaction mixture was dissolved in MeOH (20 ml), 5 ml of 5M aqueous sodium hydroxide was added, the mixture was stirred at room temperature for 1 hour and then diluted with ethyl acetate and water, and the organic layer was extracted. The organic layer was washed with a saturated saline solution, dried over sodium sulfate, filtered and then concentrated. The resultant crude product was purified by silica gel column chromatography to yield the title compound (5.1 g) as a yellow oil.

### Reference Example 4

### 5-[4-(2-hydroxypropyl)phenyl]isoxazol-3-ol

The title compound (1.84 g) was obtained as a colorless solid by the same method as in Reference Examples 1 and 2 using 1-(4-iodophenyl)propan-2-ol as a starting material.

### Reference Example 5

### 3-isopropoxyphenacylbromide

A solution of bromine (0.27 g, 1.7 mmol) in CHCl₃ (1 mL) was added to a solution of 3-isopropoxyacetophenone (0.3 g, 1.7 mmol) in CHCl₃ (5 mL) at room temperature, and the mixture was stirred overnight at room temperature. The reaction solution was concentrated, diluted with chloroform, and washed with water. The organic layer was concentrated, and the residue was purified by thin layer silica gel chromatography to give the compound of interest (100 mg).

### Reference Example 6

### N-[2-(3-isopropoxyphenyl)-1-methyethyl]-4-[3-(methoxymethoxy)isoxazol-5-yl]aniline

To a solution of 4-[3-(methoxymethoxy)isoxazol-5-yl]aniline (50 mg) and 1-(3-isopropoxyphenyl)acetone (50 mg) in Cl(CH₂)₂Cl (4 mL), Ti(i-PrO)₄ (450 mg) was added. After 1 hour, NaBH₃CN (160 mg) was added, and the mixture was stirred overnight. The reaction solution was quenched with water and filtered, followed by extracting the filtrate with ethyl acetate. The organic layer was washed with a saturated saline solution, dried over sodium sulfate, concentrated, and purified by preparative TLC to give the title compound.

### Reference Example 7

### 4-methyl-2-((1-methylethyl)oxy)pyridine

To a solution of 4-methylpyridin-2(1H)-one (3.14 g) in chloroform (30 ml), 4.03 ml of 2-iodopropane and 11.1 g of silver carbonate were added, and the reaction solution was stirred overnight at room temperature. The reaction solution was filtered through Celite, and the filtrate was concentrated under reduced pressure to yield the title compound as a pale yellow oil.

### Reference Example 8

### 1-(2-((1-methylethyl)oxy)pyridin)-4yl)propan-2-ol

To a solution of 4-methyl-2-((1-methylethyl)oxy)pyridine (1.51 g), obtained in Example 1, in tetrahydrofuran (20 ml), a 2.66 M solution of n-butyllithium in hexane (4.14 ml) was added under argon atmosphere at -78°C, and the mixture was then warmed to 0°C for 30 minutes and further stirred at 0°C for 20 minutes. The mixture was cooled to -78°C again, 0.68 ml of acetaldehyde was added, and the reaction solution was warmed to 0°C for 30 minutes. An aqueous ammonium chloride solution was added, the mixture was extracted with ethyl acetate, washed with a saturated saline solution, then dried over anhydrous magnesium sulfate, and filtered, and the filtrate was then removed under reduced pressure. The resultant residue was purified by silica gel column chromatography (eluent: ethyl acetate/hexane=10/90 to 50/50) to yield the title compound as a colorless oil.

### Reference Example 9

### 2-((1-methylethyl)oxy)-4-(2-((4-(3-(((methyloxy)methyl)oxy)isoxazol-5-yl)phenyl)oxy) propyl)pyridine

To a solution of 1-(2-((1-methylethyl)oxy)pyridine-4-yl)propan-2-ol (244 mg), obtained in Reference Example 2, 4-(3-(methoxymethoxy)-5-isoxazolyl)phenol (111 mg) and triphenylphosphine (393 mg) in tetrahydrofuran (5 ml), a 2.2 M solution of azodicarboxylic acid diethyl ester in toluene (0.68 ml) was added, and the mixture was stirred at room temperature for 30 minutes. The reaction solution was removed under reduced pressure, and the resultant residue was purified by silica gel column chromatography (eluent: ethyl acetate/hexane=10/90 to 50/50) to yield the title compound as a colorless oil.

### Reference Example 10

### 2-((1-methylethyl))oxy)-4-((2S)-2-((4-(3-(((methyloxy)methyl)oxy)isoxazo1-5-yl)pheny 1)oxy)propyl)pyridine and 2-((1-methylethyl)oxy)-4-((2R)-2-((4-(3-(((methyloxy)methyl)oxy)isoxazol-5-yl)phenyl )oxy)propyl)pyridine

Optical resolution of 2-((1-methylethyl)oxy)-4-(2-((4-(3-(((methyloxy)methyl)oxy)isoxazol-5-yl)phenyl)oxy) propyl)pyridine (194 mg), obtained in Reference Example 3, was carried out using HPLC (Chiral Pack AD-H, manufactured by Daicel Chemical Industries, Ltd.; eluent: hexane/ethanol=70/30; 0.1% diethylamine) to yield the title compound as a colorless oil.

The commercially available starting products can be used as starting materials for each example or reference example, or starting materials can be prepared by the method known in the art using commercially available products.

The usefulness of the compound according to an embodiment of the present invention was assessed for a medicament by described methods of the following tests:

### Test 1: Cloning of genes

Primers were synthesized in the domains on the opposite sides of the base sequences of the ORFs of the known GPCR and GPR120 in GenBank Accession NOs. NM 181745 (human) and NM 181748 (mouse), and the genes were cloned by RT-PCR. The base sequences of the primers used are described below. The restriction enzymes, BamHI and EcoRI, recognition sites were introduced for subcloning, respectively.
hGPR120_F01: AGGATCCGCCGCCATGTCCCCTGAATGCGCGCGGGCAG (SEQ ID NO: 1)
hGPR120_R01: CGAATTCTTAGCCAGAAATAATCGACAAGTCATTTC (SEQ ID NO: 2)
mGPR120_F01: AGGATCCGCCGCCATGTCCCCTGAGTGTGCACAGACGAC (SEQ ID NO: 3)
mGPR120_R01 : CGAATTCTTAGCTGGAAATAACAGACAAGTCATTTC (SEQ ID NO: 4)

As samples for PCR, human small intestine Marathon-ready cDNA (CLONTECH, current corporate name: TaKaRa) and cDMA obtained by reverse transcription of mouse BAT-derived RNA were used for human and mouse GPR120 receptor genes, respectively.

Using KOD Plus (TOYOBO) for PCR, 30 cycles of 94°C for 2 minutes, 94°C for 15 seconds, 55°C for 30 seconds and 68°C for 1 minute were carried out to effect reaction, follwed by addition of 0.5 units of ExTaq (TaKaRa) and incubation at 72°C for 10 minutes to carry out A-addition reaction to terminals. For mouse PCR, 35 cycles were carried out on the condition of a final DMSO concentration of 2%.

Cloning of amplified PCR products was carried out using pCR2.1-TOPO TA cloning kit (Invitrogen). For verification of base sequences, electrophoresis was carried out using BigDye Terminator Cycle Sequencing Ready Reaction Kit Ver. 3.0 and 377 DNA Sequencer (Applied Biosystems) to determine the base sequences. The human GPR120 gene was 16 amino acids shorter than the sequence registered as GenBank Accession NO. NM 181745.

The GPR120 receptor genes cloned into pCR2.1-TOPO vectors, into which the restriction enzymes, BamHI and EcoRI, recognition sites were introduced, were excised from the vectors by the enzymes and subcloned into the BamHI and EcoRI recognition sites of eucaryotic expression vector EF1/V5-His B (Invitrogen).

### Test 2: Production of expression cells

Using Lipofectamine 2000 (Invitrogen), cDNA of GPR120 receptor was transfected into CHO/NFAT-BLA cells, and drug-resistant cells were isolated to obtain GPR120 stable expression strains. The GPR120-expressed CHO cells were cultured in DMEM/F12 medium containing 10% fetal bovine serum, 100 units/ml penicillin, 0.1 mg/ml streptomycin sulfate, 250 µg/ml Zeocin, 500 µg/mL Geneticin and 15 mM HEPES.

### Test 3: Measurement of intracellular calcium concentration

On the day before the measurement day, 4µM Fluo-4 AM (fluorescence calcium indicator reagent) was incubated to be introduced into the human GPR120 expression CHO cells plated at 20000 cells per well of a 96-well black plate (ViewPlate; Packard) in the presence of 2.5 mM probenecid in a CO₂ incubator for 1 hour. To the cells was added the test compound diluted with HBSS solution containing 20 mM HEPES and 2.5 mM probenecid. Variations in the intracellular calcium concentration were measured by Fluorescence Imaging Plate Reader (FLIPR; Molecular Devices) to examine the agonist action, and EC₅₀ values were calculated.

The GPR120 agonist action of the compound groups encompassed by the compound according to an embodiment of the present invention is as follows.

**Table 5-1**

| Exemples | Agonistic activity at 10 µmol/l (%) |
|---|---|
| 1 | 104 |
| 2 | 110 |
| 3 | 105 |
| 4 | 98 |
| 5 | 94 |
| 6 | 98 |
| 7 | 98 |
| 8 | 96 |
| 9 | 100 |
| 10 | 93 |
| 11 | 91 |
| 12 | 95 |
| 13 | 93 |
| 14 | 101 |
| 15 | 97 |
| 16 | 96 |
| 17 | 97 |
| 18 | 97 |
| 19 | 96 |
| 20 | 94 |
| 21 | 95 |
| 22 | 93 |
| 23 | 101 |
| 24 | 86 |
| 25 | 91 |
| 26 | 100 |
| 27 | 99 |
| 28 | 97 |
| 29 | 102 |
| 30 | 91 |
| 31 | 93 |
| 32 | 91 |
| 33 | 84 |
| 34 | 90 |
| 35 | 98 |
| 36 | 100 |
| 37 | 97 |
| 38 | 92 |
| 39 | 90 |
| | |

**Table 5-2**

| | |
|---|---|
| 41 | 67 |
| 42 | 88 |
| 43 | 94 |
| 44 | 89 |
| 45 | 85 |
| 46 | 81 |
| 47 | 92 |
| 48 | 92 |
| 49 | 84 |
| 50 | 80 |
| 81 | 77 |
| 52 | 86 |
| 53 | 100 |
| 54 | 100 |
| 55 | 101 |
| 56 | 96 |
| 57 | 98 |
| 58 | 91 |
| 59 | 98 |
| 60 | 65 |
| 61 | 79 |
| 62 | 89 |
| 63 | 106 |
| 64 | 104 |
| 65 | 76 |

The compounds of the invention has GPR120 receptor agonist activity, and, which are useful as drugs for treating and/or preventing diabetes mellitus, obesity and hyperlipidemia.

### Industrial

### INDUSTRIAL APPLICABILITY

The compounds of the invention or the pharmaceutically acceptable salt thereof act as GPR120 receptor function regulating agents, specially GPR120 receptor agonist activity, and which are useful as drugs for treating and/or preventing diabetes mellitus, or hyperlipidemia.

## Claims

1. A compound represented by formula (I): 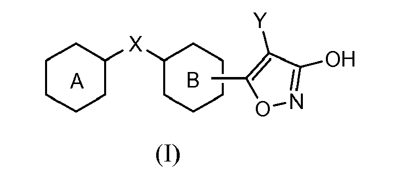 or a pharmaceutically acceptable salt thereof, wherein: 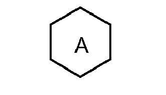 represents phenyl, 5- or 6-membered heteroaryl, or fused ring with phenyl or 5- or 6-membered heteroaryl
said phenyl, 5- or 6-membered heteroaryl, or fused ring with phenyl or 5- or 6-membered heteroaryl, is optionally substituted with 1-4 same or different groups selected from the group consisting of:
lower alkoxy optionally substituted 1-3 halogen atoms,
lower alkoxycarbonyl,
lower alkenyloxy,
lower alkynyloxy,
lower alkylthio optionally substituted with 1-3 halogen atoms,
halo(lower)alkyloxy, cycloalkyloxy,
lower alkyl optionally substituted with 1-3 halogen atoms,
lower alkenyl,
lower alkynyl,
cycloalkylthio,
lower alkylamino,
lower alkenylamino,
lower alkynylamino,
lower haloalkylamino,
cycloalkylamino,
nitro,
halogen,
cyano,
lower alkylsulfonyl,
lower alkenylsulfonyl,
lower alkynylsulfonyl,
phenoxy,
phenyl,
5- or 6-membered heteroaryloxy,
5- or 6-membered heteroaryl,
5- or 6-membered heteroarylalkyloxy, and
Arylalkyloxy; 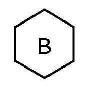 represents a divalent group, in which two hydrogen atoms are removed from a benzene, pyridine, pyrazine, pyrimidine or pyridazine ring, and which
is optionally substituted with 1-4 same or different groups selected from the group consisting of:
halogen,
lower alkyl, and
lower alkoxy;
X is lower alkylene having a main chain including 2-4 carbon atoms, wherein one or
two of the carbon atoms constituting the main chain is optionally substituted with oxygen, sulfur or nitrogen,
said lower alkylene is substituted with 1-3 same or different groups selected from the group consisting of:
(1) amino optionally substituted with lower alkyl, lower alkylsulfonyl and lower alkanoyl,
(2) halogen,
(3) lower alkenyl,
(4) lower alkyl, when identical carbon atoms constituting the lower alkylene are substituted with two lower alkyl groups, the lower alkyl groups may together form 3- to 7-membered aliphatic rings , and one or two of carbon atoms constituting the aliphatic rings is optionally substituted with nitrogen, sulfur or oxygen,
(5) lower alkynyl,
(6) lower alkylidene,
(7) imino,
(8) arylalkyl,
(9) 5- or 6-membered heteroarylalkyl having 1-4 hetero atoms selected from the group consisting of nitrogen, sulfur and oxygen atoms in a ring,
(10) heteroaryl having 1-4 hetero atoms selected from the group consisting of nitrogen, sulfur and oxygen atoms in a ring,
(11) arylalkyloxy, and
(12) 5- or 6-membered heteroarylalkyloxy having 1-4 hetero atoms selected from the group consisting of nitrogen, sulfur and oxygen atoms in a ring;
said lower alkyl, lower alkynyl and lower alkylidene is optionally substituted with 1-3 halogen atoms or hydroxy or lower alkoxy groups;
said imino is optionally substituted with 1-3 lower alkyl, lower alkylsulfonyl, cyano,
nitro and lower alkanoyl groups;
said lower alkylene may have one or two double or triple bonds in a main chain and, in addition, the lower alkylene is optionally substituted with 1-3 lower alkoxy, oxo or
hydroxy groups;
X together with 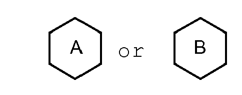 may form 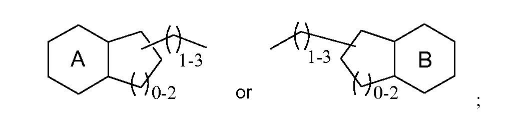 one or two of carbon atoms constituting 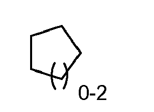 is optionally substituted with nitrogen, sulfur or oxygen and have one or two double bonds in a ring;
one carbon atom constituting 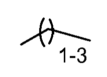 is optionally substituted with nitrogen, sulfur or oxygen; and
Y represents hydrogen, lower alkyl optionally substituted with 1-3 same or different lower alkoxy groups and halogen atoms, lower alkoxy or halogen.

2. The compound according to claim 1, or the pharmaceutically acceptable salt thereof, wherein the formula (I) is formula (I-1): 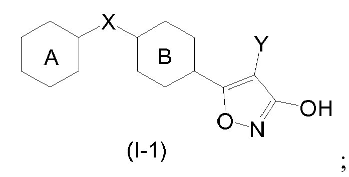 and
Y is a hydrogen atom.

3. The compound according to claim 2, or the pharmaceutically acceptable salt thereof, wherein X is lower alkylene in which a main chain contains three carbon atoms and one or two of carbon atoms constituting the main chain are substituted with oxygen, sulfur or nitrogen; and
said lower alkylene is substituted with 1-3 same or different groups selected from the group consisting of lower alkyl, halogen and oxo.

4. The compound according to claim 2, or the pharmaceutically acceptable salte thereof, wherein X is lower alkylene in which a main chain contains three carbon atoms and one or two of carbon atoms constituting the main chain are substituted with oxygen, sulfur or nitrogen;
said lower alkylene is substituted with 1-3 same or different groups selected from the group consisting of lower alkyl, halogen and oxo; and 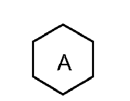 is phenyl or pyridinyl optionally substituted with 1-2 same or different groups selected from the group consisting of:
lower alkoxy optionally substituted with 1-3 same or different halogen atoms, C₃₋₇ cycloalkyloxy,
lower alkyl optionally substituted with 1-3 same or different halogen atoms,
lower alkenyl and halogen atoms.

5. The compound according to claim 2, or the pharmaceutically acceptable salt thereof, wherein, X is lower alkylene in which a main chain contains three carbon atoms or one or two of carbon atoms constituting the main chain are substituted with oxygen, sulfur or nitrogen;
said lower alkylene is substituted with 1-3 same or different groups selected from the group consisting of:
lower alkyl,
halogen and oxo; 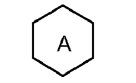 is phenyl or pyridinyl optionally substituted with 1-2 same or different groups selected from the group consisting of:
lower alkoxy optionally substituted with 1-3 same or different halogen atoms, C₃₋₇ cycloalkyloxy,
lower alkyl optionally substituted with 1-3 same or different halogen atoms,
lower alkenyl and halogen atoms; and 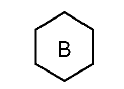 represents a divalent group, in which two hydrogen atoms are removed from a benzene or pyridine ring,
said ivalent group is optionally substituted with 1-4 same or different groups selected from the group consisting of:
halogen,
lower alkyl, and
lower alkoxy.

6. The compound according to claim 5, or the pharmaceutically acceptable salt thereof, wherein: 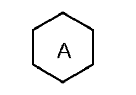 represents a group selected from the group consisting of:

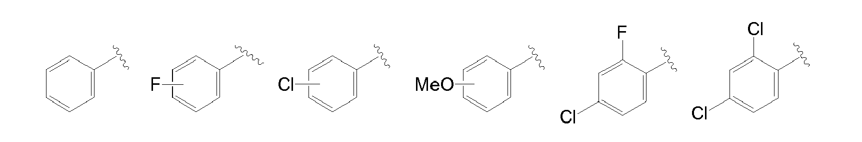

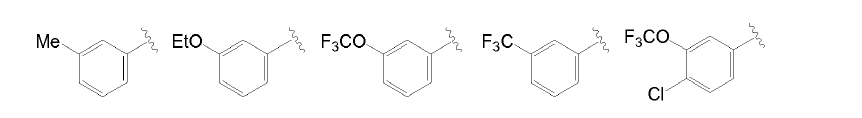

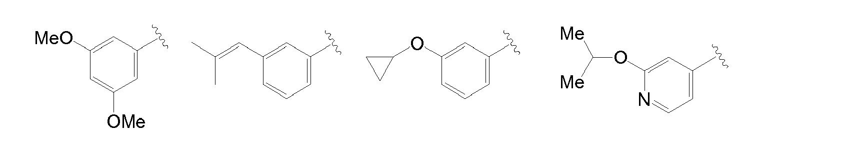

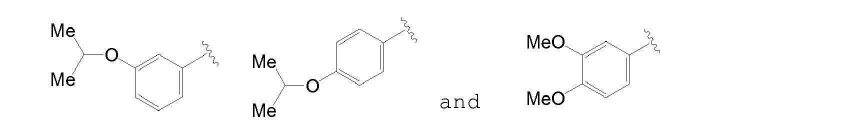

7. The compound according to claim 5, or the pharmaceutically acceptable salt thereof, wherein: 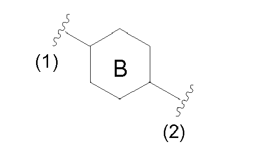 is a group selected from the group consisting of: 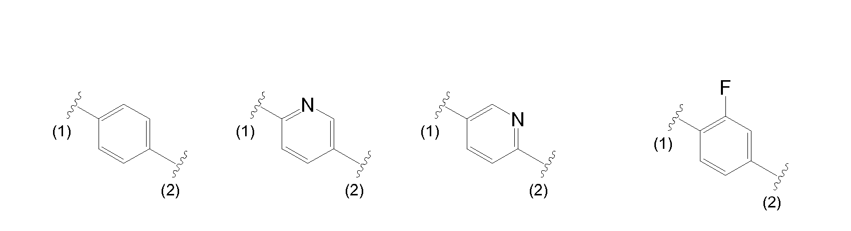 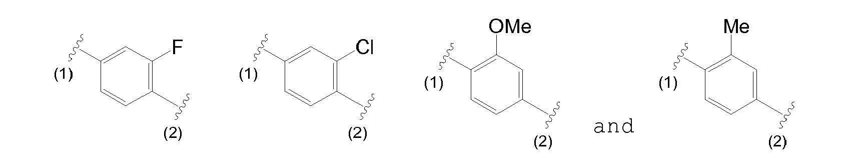 wherein 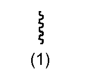 represents a binding site with X; and 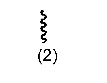 represents a binding site with 3-hydroxy-isoxazolyl.

8. The compound according to any one of claims 1 to 7, or the pharmaceutically acceptable salt thereof, wherein X is lower alkylene in which a main chain contains three carbon atoms or one of carbon atoms constituting the main chain is substituted with an oxygen, sulfur or nitrogen atom; and
the lower alkylene is substituted with one or two same or different groups selected from the group consisting of lower alkyl, halogen and oxo.

9. The compound according to any one of claims 1 to 7, or the pharmaceutically acceptable salt thereof, wherein X is a group selected from the group consisting of: 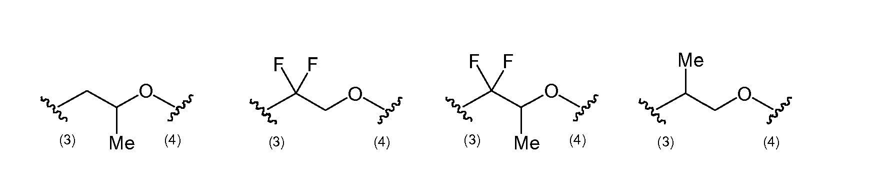 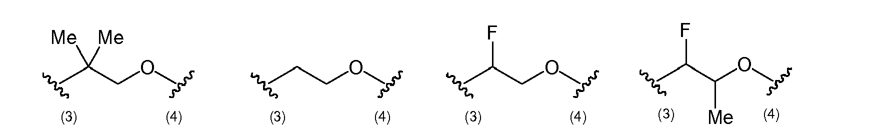 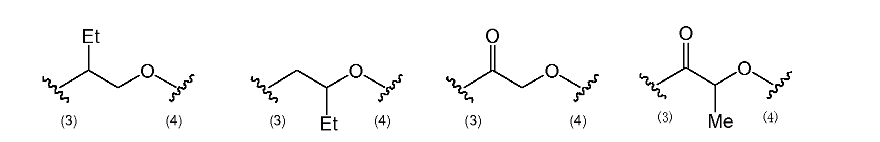 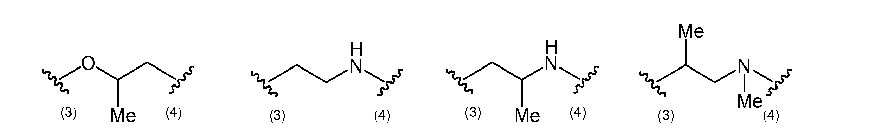 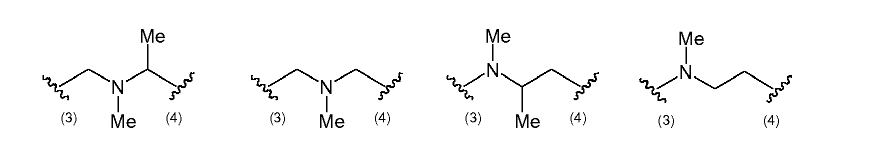 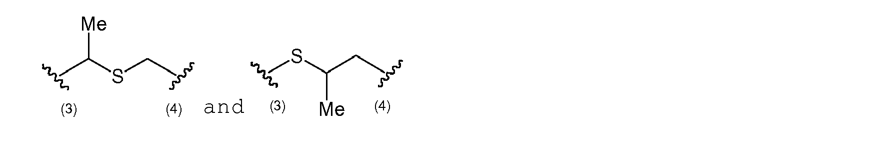 wherein 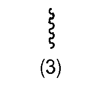 represents a binding site with 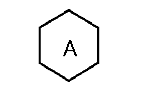 ; and 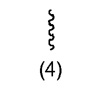 represents a binding site with

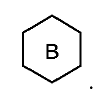

10. The compound according to claim 1, or the pharmaceutically acceptable salt thereof, wherein the compound represented by the formula (I) is:
5-(4-(2-(3-isopropoxyphenyl)-1-methylethoxy)phenyl)isoxazol-3-ol,
5-(4-(2-fluoro-2-(3-methoxyphenyl)ethoxy)phenyl)isoxazol-3-ol,
5-(4-(2,2-difluoro)-2-(3-methoxyphenyl)ethoxy)phenyl)isoxazol-3-ol,
5-(4-(2-(3-isopropoxyphenyl)propoxy)phenyl)-isoxazol-3-ol,
5-(4-(2-methyl-2-(3-isopropoxyphenyl)propoxy)phenyl)-isoxazol-3-ol,
5-{2-fluoro-4-[2-(3-isopropoxyphenyl)-1-methylethoxy]phenyl}isoxazol-3-ol,
5-(6-(2-(3-isopropoxyphenyl)-1-methylethoxy)pyridin-3-yl)isoxazol-3-ol,
5-(5-(2-(3-isopropoxyphenyl)-1-methylethoxy)pyridin-2-yl)isoxazol-3-ol,
5-(4-(((3-isopropoxybenzyl)(methyl)amino)methyl)phenyl)isoxazol-3-ol,
5-(4-(2-phenoxypropyl)phenyl)isoxazol-3-ol,
5-(4-(2-(3-methylphenoxy)propyl)phenyl)isoxazol-3-ol,
5-(4-(2-(3-methoxyphenoxy)propyl)phenyl)isoxazol-3-ol,
5-(4-(2-(3-(trifluoromethyl)phenoxy)propyl)phenyl)isoxazol-3-ol,
5-(4-(2-(4-methylphenoxy)propyl)phenyl)isoxazol-3-ol,
5-(4-(2-(3,5-dimethoxyphenoxy)propyl)phenyl)isoxazol-3-ol,
5-(4-(2-(4-chlorophenoxy)propyl)phenyl)isoxazol-3-ol,
5-(4-(2-(4-chloro-2-fluorophenoxy)propyl)phenyl)isoxazol-3-ol,
5-(4-(2-(2,4-dichlorophenoxy)propyl)phenyl)isoxazol-3-ol,
5-(4-(2-(3((trifluoromethyl)oxy)phenyl)oxy)propyl)phenyl)isoxazol-3-ol,
5-{4-[((1R)-1-methyl-2-{3-[(1-methylethyl)oxy]phenyl}ethyl)oxy]phenyl}isoxazol-3-ol ,
5-{4-[((1S)-1-methyl-2-{3-[(1-methylethyl)oxy]phenyl}ethyl)oxy]phenyl}isoxazol-3-ol ,
5-[4-({(1R)-1-methyl-2-[3-(cyclopropyloxy)phenyl]-1-methylethyl}oxy)phenyl]isoxazo 1-3-ol,
5-{4-[((1R)-1-methyl-2-{3-[(trifluoromethyl)oxy]phenyl}ethyl)oxy]phenyl}isoxazol-3-ol, 5-{4-[((1R)-1-methyl-2-{4-[(trifluoromethyl)oxy]phenyl}ethyl)oxy]phenyl}isoxazol-3-ol,
5-{4-[((1R)-2-{4-chloro-3-[(trifluoromethyl)oxy]phenyl}-1-methylethyl)oxy]phenyl}is oxazol-3-ol, 5- {6-[((1R)-1-methyl-2-{3-[(1-methylethyl)oxy]phenyl}ethyl)oxy]pyridin-3-yl}isoxazo 1-3-ol hydrochloride, 5-{6-[((1S)-1-methyl-2-{3-[(1-methylethyl)oxy]phenyl}ethyl)oxy]pyridine-3-yl}isoxaz ol-3-ol hydrochloride, 5-{2-fluoro-4-[((1R)-1-methyl-2-{3-[(1-methylethyl)oxy]phenyl}ethyl)oxy]phenyl}isox azol-3-ol, 5-{4-[(2-fluoro-2-{3-[(1-methylethyl)oxy]phenyl}ethyl)oxy]phenyl}isoxazol-3-ol, 5-{4-[(2,2-difluoro-2-{3-[(1-methylethyl)oxy]phenyl}ethyl)oxy]phenyl}isoxazol-3-ol, 5-{4-[(2-fluoro-2-{3-[(1-methylethyl)oxy]phenyl}ethyl)oxy]phenyl}isoxazol-3-ol, 5-{2-[((1R)-1-methyl-2-{3-[(1-methylethyl)oxy]phenyl}ethyl)oxy]pyrimidin-5-yl}isoxa zol-3-ol, 5-(4-(2-(3-isopropoxyphenyl)propoxy)phenyl)-isoxazol-3-ol, 5-(4-(2-(3-isopropoxyphenyl)-1-ethylethoxy)phenyl)isoxazol-3-ol, 5-(4-(2-(3-ethylphenyl)-1-methylethoxy)phenyl)isoxazol-3-ol, 5-(4-(2,2-difluoro-2-(3-isopropoxyphenyl)ethoxy)phenyl)isoxazol-3-ol, 5-{2-fluoro-4-[2-(3-ethoxyphenyl)-l-methylethoxy]phenyl}isoxazol-3-ol, 5-{2-fluoro-4-[2-(3-isopropoxyphenyl)-2-methylpropoxy]phenyl}isoxazol-3-ol, 5-{2-fluoro-4-[2-(3-isopropoxyphenyl)propoxy]phenyl}isoxazol-3-ol, 5-(6-(2-(3-isopropoxyphenyl)propoxy)pyridin-3-yl)isoxazol-3-ol, 5-(4-{[[1-(3-isopropoxyphenyl)ethyl](methyl)amino]methyl}phenyl)isoxazol-3ol, 2-[4-(3-hydroxyisoxazol-5-yl)phenoxy]-1-(3-isopropoxyphenyl)ethanone, 2-[2-(3-ethoxyphenyl)-1-methylethoxy]-5-(3-hydroxyisoxazol-5-yl)pyridinium trifluoroacetate, 5-(3-hydroxyisoxazol-5-yl)-2-[2-(3-isopropoxyphenyl)-2-methylpropoxy]pyridinium trifluoroacetate,
3-{2-[4-(3-hydroxyisoxazol-5-yl)phenoxy]propyl}-N,N-dimethylaniline hydrochloride,
2-[4-(3-hydroxyisoxazol-5-yl)phenoxy]-1-(3-isopropoxyphenyl)propan-1-one,
5-(4-{[1-(3-isopropoxyphenyl)ethoxy]methyl}phenyl)isoxazol-3-ol,
N-{2-[4-(3-hydroxyisoxazol-5-yl)phenyl]ethyl}-3-isopropoxy-N-methylaniline trifluoroacetate,
1-[4-(3-hydroxyisoxazol-5-yl)phenyl]-N-(3-isopropoxybenzyl)-N-methylethanamine trifluoroacetate,
5-[4-({[1-(3-isopropoxyphenyl)ethyl]thio}methyl)phenyl]isoxazol-3-ol, N-[4-(3-hydroxyisoxazol-5-yl)benzyl]-3-isopropoxy-N-methylbenzenesulfonamide,
5-{4-[2-(3-isopropoxyphenoxy)propyl]phenyl}isoxazol-3-ol,
5-{4-[2-(3-isobutylphenyl)-1-methylethoxy]phenyl}isoxazol-3-ol,
5-{3-fluoro-4-[2-(3-isopropoxyphenyl)-1-methylethoxy]phenyl}isoxazol-3-ol,
5-(4-{2-[(3-isopropoxyphenyl)thio]propyl}phenyl)isoxazol-3-ol,
5-{4-[2-(3-ethoxyphenyl)-1-methylethoxy]-3-fluorophenyl}isoxazol-3-ol,
5-{4-[2,2-difluoro-2-(3-isopropoxyphenyl)ethoxy]-2-fluorophenyl}isoxazol-3-ol,
5-(4-{2-[(3-isopropoxyphenyl)(methyl)amino]propyl}phenyl)isoxazol-3-ol,
4-(3-hydroxyisoxazol-5-yl)-N-[2-(3-isopropoxyphenyl)-1- -methylethyl]aniline trifluoroacetate,
4-(3-hydroxyisoxazol-5-yl)-N-[2-(3-isopropoxyphenyl)ethyl]-N-methylaniline trifluoroacetate,
5-{4-[2-(3-isopropoxyphenyl)-1-methylethoxy]-3-methoxyphenyl}isoxazol-3-ol,
5-(4-((1-methyl-2-(2-((1-methylethyl)oxy)pyridin-4-yl)ethyl)oxy)phenyl)isoxazol-3-ol hydrochloride,
5 -(4-(((1S)-1-methyl-2-(2-((1-methylethyl)oxy)pyridin-4-yl)ethyl)oxy)phenyl)isoxazol-3-ol sodium,
5-(4-(((1R)-1-methyl-2-(2-((1-methylethyl)oxy)pyridin-4-yl)ethyl)oxy)phenyl)isoxazol-3-ol sodium or
N-[4-(3-hydroxyisoxazol-5-yl)benzyl]-3-isopropoxy-N-methylaniline.

11. A GPR120 function regulating agent comprising the compound according to any one of claims 1 to 10 or the pharmaceutically acceptable salt thereof, as an active ingredient.

12. A GPR120 agonist comprising the compound according to any one of claims 1 to 10 or the pharmaceutically acceptable salt thereof, as an active ingredient.

13. A pharmaceutical composition comprising the compound according to any one of claims 1 to 10 and a pharmaceutically acceptable carrier.

14. An agent for treating diabetes mellitus, obesity or hyperlipidemia, comprising the compound according to any one of claims 1 to 10 as an active ingredient.
